# EUROPEAN PATENT SPECIFICATION

(11) **EP 0 485 591 B1**
(45) Date of publication and mention of the grant of the patent: **06.11.1996**
(21) Application number: 91912336.4
(22) Date of filing: 05.06.1991
(51) Int. Cl.: C12N 15/00, C12N 1/20, C12R 1/63, A61K 39/106

(54) **METHOD FOR ISOLATING DELETION MUTANTS OF VIBRIO CHOLERAE AND CULTURES CONTAINING VIBRIO CHOLERAE**
VERFAHREN ZUM ISOLIEREN VON VIBRIO CHOLERAE-DELETIONSMUTANTEN UND VIBRIO CHOLERAE ENTHALTENDE KULTUREN
PROCEDE SERVANT A ISOLER DES MUTANTS DE DELETION DE VIBRIO CHOLERAE ET CULTURES CONTENANT VIBRIO CHOLEREA

(30) Priority: 05.06.1990 US 533315
(43) Date of publication of application: 20.05.1992
(73) Proprietor: THE UNIVERSITY OF MARYLAND AT BALTIMORE, Baltimore Maryland 21201 (US)
(72) Inventor: KAPER, James, B., Columbia, MD 21045 (US); BAUDRY-MAURELLI, Bernadette, Silver Spring, MD 20901 (US); FASANO, Alessio, I-84100 Salerno (IT)
(74) Representative: Grünecker, Kinkeldey, Stockmair & Schwanhäusser Anwaltssozietät
(86) International application number: US9103812
(87) International publication number: WO9118979

(56) References cited:
- EP-A- 0 119 031
- US-A- 4 882 278
- INFECTION AND IMMUNITY vol. 56, no. 1, January 1988, WASHINGTON US pages 161 - 167 LEVINE ET AL. 'Volunteer studies of deletion mutants of Vibrio cholerae O1 prepared by recombinant techniques'
- PROCEEDINGS OF THE NATIONAL ACADEMY OF SCIENCES OF USA. vol. 84, May 1987, WASHINGTON US pages 2833 - 2837 TAYLOR ET AL. 'Use of phoA gene fusions to identify a pilus colonization factor coordinately regulated with cholera toxin'
- NATURE. vol. 306, no. 5943, 8 December 1983, LONDON GB pages 551 - 557 MEKALANOS ET AL. 'Cholera toxin genes: nucleotide sequence deletion analysis and vaccine development'
- LANCET, issued 27 August 1988, M.M. LEVINE et al., "Safety. Immunogenicity and Efficacy of Recombinant Live Oral Cholera Vaccines. CVD103 and CVD 103HgR". pages 467-470.
- GENE, Vol. 39, issued 1985, B.D. GAMBILL et al., "Versatile Mercuryresistant cloning and expression vectors", pages 293-297

## Description

This application is a continuation-in-part of applicant's U.S. Patent application 06/581,406 filed February 17th, 1984, which is a continuation-in-part of U.S. Patent application 06/472,276, filed March 4th, 1983, which are incorporated herein by reference. This application is also a continuation-in-part of applicant's U.S. Patent Application 07/363,383, filed June 5th, 1989 and allowed December 12th, 1989, which is a continuation of U.S. Patent Application 06/867,633, filed May 27th, 1986, which is a continuation of U.S. Patent Application 06/472,276, filed March 4th, 1983, which are incorporated herein by reference. The research outlined in this application was supported by the National Institute of Health and in part by the Medical Biotechnology Center of the Maryland Biotechnology Institute.

### BACKGROUND OF THE INVENTION

Vibrio cholerae (V.cholerae) is a non-invasive enteropathogen of the small bowel that does not penetrate the mucosal surface. Local SIgA mediated immunity at the mucosal surface is therefore implicated as a protective mechanism. Pathogenic V. cholerae 01 elaborate a protein enterotoxin (also know as cholera enterotoxin, or choleragen, or cholera toxin) which is responsible for induction of copious secretion by the intestine resulting in watery diarrhea, the clinical consequence of cholera infection. The genes responsible for cholera toxin are the ctx genes (also known as the tox genes). Cholera diarrhea can be extraordinarily severe and result in loss of so much body water and salts that dehydration, acidosis, shock, and death ensue without prompt therapy. Applicants have discovered that a second enterotoxin is produced by V. cholerae called zonula occludens toxin, reported in Fasano et al, Vibrio cholerae Produces a Second enterotoxin Which Affects Intestinal Tight Junctions, Proc. Natl. Acad. Sci. USA 88:5242-5246. Further, there is a region of the chromosome containing the ctx genes which contains multiple copies of a 2700 base pair sequence called RS1 (for repetitive sequence). Mekalanos, Cell 35, 253- 263 (1983).

The cholera vaccines that have been developed can be broadly divided into two categories; those aiming to stimulate antitoxic immunity and those intending to induce antibacterial immunity. Experiments with animal models support a protective role for either or both antitoxic and antibacterial immunity. It has been suggested that when both types of immunity work in unison, there is a synergistic effect. [Holmgren, J. et al. J. Infect. Dis. 136 Suppl., S105-S1122 (1977); Peterson, J.W. Infect. Immun. 26, 594 (1979); Resnick, I.G. et al. Infect. Immun. 13, 375 (1980); Svennerholm, A.-M. et al. Infect. Immun. 13, 735 (1976)]. However, it appears that protective immunity in humans can be conferred without such synergistic effect, that is by either antitoxic immunity or antibacterial immunity [Eubanks, E.R. et al. Infect. Immun. 15, 533 (1977); Fujita, K. et al. J. Infect. Dis. 125, 647 (1972); Holmgren, J., J. Infect. Dis., supra; Lange, S. et al. Acta Path. Microbiol. Scand Sect. C 86, 145 (1978); Peterson, J.W., supra (1979); Pierce, N.F. et al. Infect Immun. 37, 687 (1982); Pierce, N.F. et al. Infect. Immun. 21, 185 (1978); Pierce, N.F. et al. J. Infect. Dis. 135, 888 (1977); Resnick, I.G. et al., supra; Svennerholm, A.-M. et al, supra].

### KILLED WHOLE CELL VACCINES

### 1. Parenteral Whole Cell Vaccines

For almost a century, killed whole V. cholerae have been employed as parenteral vaccines; these vaccines are still commercially available. Experience with the parenteral whole cell vaccines has been reviewed recently in Joo, I. "Cholera Vaccines." In Cholera. (Barua D. and Burrows W., eds.), Saunders, Philadelphia, pp. 333-355 (1974) and in Feeley, J.D. et al. In Cholera and Related Diarrheas. 43rd Nobel Symp., Stockholm 1978. (O. Oucherlong, J. Holmgren, eds.) Karger, Basel, pp. 204-210 (1980). Such vaccines stimulate high titers of serum vibrioicidal antibodies. They also stimulate increases in intestinal SIgA antibody to V. cholerae somatic O antigen when given to Pakistanis but not to Swedes [Svennerholm, A.-M. et al. Infect. Immun. 30., 427 (1980); Svennerholm, A.-M. et al. Scan. J. Immun. 6, 1345 (1977)]. It has been suggested that the Pakistani vaccine recipients respond in this way because they are already immunologically primed from prior antigenic contact, while persons living in a non-endemic area (e.g., Sweden) are not. In field trials parenteral killed whole cell vaccines have been shown to confer significant protection against the homologous V. cholerae serotype, but usually for a period of less than one year [Joo, I. supra; Feeley, J.C. , supra; Svennerholm, A.-M. et al. supra, (1980); Svennerholm, A.-M. et al. supra, (1977); Mosley, W.H. et al. Bull. Wld. Hlth. Org. 49, 13 (1973); Phillipines Cholera Committee, Bull. Wld. Hlth. Org. 49, 381 (1973)]. There is some evidence to suggest that parenteral whole cell Inaba vaccine provides good, short term protection against Ogawa, as well as against Inaba cholera, while Ogawa vaccine is effective only against Ogawa.

By use of adjuvants, it has been possible to maintain a vaccine efficacy of approximately 70% for up to one-and-one-half years with parenteral vaccine (see, e.g., Saroso, J.S. et al. Bull. Wld. Hlth. Org. 56, 619 (1978)). However, the adverse reactions encountered at the site of inoculation with adjuvanted vaccines (which include sterile abscesses) are sufficiently frequent and severe to preclude routine use of such adjuvanted vaccines.

### 2. Oral Whole Cell Vaccines

Killed whole vibrios administered orally stimulate the appearance of local intestinal antivibrio antibody. [Freter, R. J. Infect Dis. 111, 37 (1972); Freter R. et al. J. immunol. 91 724 (1963); Ganguly, R. et al. Bull. Wld. Hlth. Org. 52, 323 (1975)]. Other investigators have shown substantial vaccine efficacy, but a large proportion of the vaccines developed diarrhea after subsequent challenge with pathogenic vibrios [Cash, R.A. et al. J. Infect. Dis. 130, 325 (1974)].

### TOXOIDS

Immunizing agents intended to prevent cholera by means of stimulating antitoxic immunity include:
1) Formaldehyde-treated cholera toxoid
2) Glutaraldehyde-treated cholera toxoid;
3) Purified B subunit; and
4) Procholeragenoid (with or without formaldehyde treatment).

### 1. Formaldehyde-Treated Cholera Toxoid

Treatment of purified cholera toxin in vitro with formaldehyde eradicates its toxicity, resulting in a toxoid that exhibits little toxic biological activity but stimulates antitoxic antibodies following parenteral immunization of animals. However, when the first toxoid of this type was administered to monkeys and man as a parenteral vaccine, the toxoid reverted to partial toxicity causing unacceptable local adverse reactions at the site of inoculation [Northrup, R.S. et al. J. Infect. Dis. 125, 471 (1972)]. An aluminum-adjuvanted formalinized cholera toxoid has been administered parenterally to Bangladeshi volunteers, including lactating mothers, but no field trials with this vaccine have been undertaken [Merson, M.H. et al. Lancet I, 931 (1980)]. Formalinized cholera toxoid prepared in the presence of glycine has also been tried by the parenteral route, but the vaccine showed no evidence of efficacy [Ohtomo, N. In Proceedings of the 12th Joint Conference on Cholera, U.S.-Japan Cooperative Medical Science Program, Sapporo (Fukumi H., Zinnaka Y., eds.) pp. 286-296 (1976); Noriki, H. In Proceedings of the 12th Joint Conference on Cholera, U.S.-Japan Cooperative Medical Science Program, Sapporo (Fukumi H., Zinnaka Y., eds.) pp. 302-310 (1976)].

### 2. Glutaraldehyde-Treated Cholera Toxoid

Methods have been developed for the large-scale preparation of a glutaraldehyde-treated cholera toxoid that is essentially free of contaminating somatic antigen [Rappaport, E.S. et al. Infect. Immun. 14, 687 (1976)]. It was hoped that this antigen could be used to assess in a "pure" manner the protective role of antitoxic immunity alone. A large-scale field trial of this toxoid given as a parenteral vaccine was carried out in Bangladesh in 1974 [Curlin, G. et al. In Proceeding of the 11th Joint Conference on Cholera, U.S.-Japan Cooperative Medical Science Program. pp. 314-329, New Orleans, (1975)]. The toxoid stimulated high titers of circulating antitoxins in Bangladeshi recipients. Two waves of cholera, El Tor Inaba followed by El Tor Ogawa, struck the field area allowing a fair evaluation of vaccine efficacy. A protective effect could be demonstrated in only one age group and was restricted to the period of the Inaba epidemic, so that glutaraldehyde-treated cholera toxoid given alone as a parenteral vaccine provided little protection and was substantially inferior to similar field trials in the same population with parenteral killed whole cell vaccines.

The use of glutaraldehyde-treated cholera toxoid as an oral vaccine has been investigated on the assumption that toxoid given by this route might be more efficient by stimulating intestinal antitoxin [Levine, M.M. et al. Trans. Rov. Soc. Trop. Med. Hyg. 73, 3, (1979)]. Two groups of volunteers were immunized with three 2.0 mg., or three 8.0 mg doses of toxoid given directly into the small intestinal lumen (via intestinal tube) at monthly intervals. The vaccines and unimmunized controls then participated in experimental cholera challenge studies. In neither challenge study was the attack rate or severity of diarrhea significantly diminished in the vaccines when compared with controls. The lack of efficacy of oral glutaraldehyde-treated cholera toxoid may be due to the fact that the capacity of B subunits to bind to GM1 ganglioside is greatly diminished as a consequence of toxoiding with glutaraldehyde.

### 3. Purified B Subunit

Cholera enterotoxin is composed of two subunits designated A and B. The A subunit induces the enzymatic changes which lead to fluid secretion, while the non-toxic B subunit is the immunogenic moiety that binds to the receptor for toxin (GM1 ganglioside) on intestinal epithelial cells [Holmgren, J. Nature 292, 413 (1981)]. It has been shown that purified B subunit given either orally or parenterally to Bangladeshis stimulates the appearance of SIgA antitoxin in intestinal fluid, a result attributable to immunological priming in a cholera-endemic area [Svennerholm, A.-M. et al. Lancet I, 305 (1982)].

The major advantages of B subunit oral vaccine to stimulate antitoxic immunity include its complete safety (there is not potential for reversion to toxin as exists with toxoids) and retention of its capacity to adhere to toxin receptors on enterocytes. Animal studies suggest that it is less potent than native holotoxin in stimulating antitoxin [Pierce, N.F. supra, (1982)].

It will be understood that the purified B subunit can be used, if at all, in conjunction with e.g. oral killed vibrios as a combination oral vaccine intended to stimulate both antibacterial and antitoxic antibodies.

### 4. Procholeragenoid

Procholeragenoid is the large molecular weight toxoid (ca. 1,000,000 MW) that results when cholera enterotoxin is heated at 65°C for at least five minutes [Finkelstein, R.A. et al. J. Immunol. 107, 1043 (1971)]. It is immunogenic while retaining less that 5% of the biological toxic activity of the parent toxin. Heating for longer times (e.g., 25 minutes) produces less biological toxicity [Germanier, R. et al. Infect. Immul 13, 1692 (1976)], and subsequent treatment with formaldehyde completely abolishes residual biological toxicity. The resultant formaldehyde-treated procholeragenoid is at least as potent as the parent toxin in stimulating serum antitoxin following immunization of rabbits. Swiss volunteers developed brisk serum antitoxin responses following parenteral immunization with 10, 30, or 100 mcg doses of formaldehyde-treated procholeragenoid [Germanier, R. et al. J. Infect. Dis. 135. 512 (1977)]. No notable adverse reactions were observed.

As an oral antigen procholeragenoid is more immunogenic when given in the form without formaldehyde-treatment. In dogs, untreated procholeragenoid is tolerated as well as an oral vaccine; oral doses (with NaHCO₃) up to 500 mcg do not case diarrhea. Five 500 mcg doses spaced over 42 days stimulate significant protection in dogs against oral challenge with pathogenic V. cholerae. Doses of 50 mcg and 200 mcg with NaHCO₃ have been given to groups of six and four adult volunteers, respectively, without eliciting adverse reactions.

It will be understood that procholeragenoid can be used in conjunction with e.g. killed vibrios or other relevant antigens capable of stimulating antibacterial immunity so that the antitoxic immunity induced by procholeragenoid is enhanced.

### COMBINATION VACCINES

The major attraction of non-living, oral cholera vaccine is its safety. An oral vaccine consisting of a combination of antigens, intending to stimulate both antibacterial and antitoxic immunity, would be most likely to succeed for the following reasons: Toxoid vaccines that stimulate purely antitoxic immunity have not been shown to be efficacious in protecting man against cholera, although they may protect animal models. In addition, oral or parenteral killed whole cell vaccines that stimulate no antitoxic immunity provide significant protection against cholera in man, albeit for a short period of time. Furthermore, combinations of antigens (such as crude cholera toxin, or toxin plus lipopolysaccaride) that stimulate both antitoxic and antibacterial immunity, give synergistic protection.

Two studies so far have been carried out in many with combination vaccines. In the first, nine volunteers who ingested glutaraldehyde-treated cholera toxoid (2 mg weekly for four weeks) plus killed El Tor Inaba vibrios (10¹⁰ vibrios twice weekly for four weeks) Were challenged after one month with 10⁶ pathogenic El Tor Inaba vibrios, along with six unimmunized controls. Diarrhea occurred in only two of nine vaccines, versus four of six controls (vaccine efficacy 67%) and illness was clearly attenuated in the two ill vaccines. More pertinent, perhaps, is the observation that V. cholerae could be directly cultured from stools of only two of nine vaccines, versus six of six controls. This demonstrates that immunologic mechanisms impeded the proliferation of vibrios.

More recently, three doses of B subunit/killed whole cell vaccine was given to adult volunteers who participated in a vaccine efficacy challenge. The combination vaccine was give on days 0, 14, and 28. Each of the three doses of vaccine contained 0.5 mg of purified B subunit and 2 x 10¹¹ killed V. cholerae (5 x 10¹⁰ classical Inaba, 5 x 1010 classical Ogawa, and 1 x 10¹¹ El Tor Inaba).

A group of eleven volunteers immunized with this combination vaccine were challenged one month after their last dose with 10⁶ pathogenic V. cholerae El Tor Inaba, along with seven control volunteers. Diarrhea occurred in seven of seven controls, but in only four of eleven vaccines (p=0.01). The illness in the four vaccines was definitely milder.

Thus, results of studies with oral toxoid/killed whole cell vaccine combinations demonstrate a measurable degree of efficacy. The protective vaccine efficacy, however, is only moderate (55-65%) and multiple doses are required to induce the protection.

### ATTENUATED V. CHOLERAE VACCINES

Both classical and El Tor clinical cholera infections stimulate a high degree of protective immunity for at least three years in North American volunteers [Cash, R.A. et al., supra (1974); Levine, M.M. et al. , supra (1979); Levine, M.M. et al. "Volunteers studies in development of vaccines against cholera and enterotoxigenic Escherichia coli: a review," in Acute Enteric Infections in Children: New Prospects for Treatment and Prevention. (T. Holm, J. Holmgren, M. Merson, and R. Mollby, eds.) Elsevier, Amsterdam, pp. 443-459 (1981); and Levine, M.M. et al. J. Infect. Dis. 143, 818 (1981)]. Based on these observations in volunteers, perhaps the most promising approach toward immunologic control of cholera may be with attenuated nontoxigenic V. cholerae strains employed as oral vaccines.

### 1. Naturally-Occurring Strains

Non-toxigenic V. cholerae 01 strains isolated from environmental sources in India and Brazil have been evaluated in volunteers as potential vaccine candidates with disappointing results. They either failed to colonize the intestine of man, or did so minimally; vibrocidal antibody responses were meager, and they failed to provide protection in experimental challenge studies [Cash, R.A. et al. Infect. Immun. 10, 762 (1974); Levine M.M. et al. J. Infect. Dis. 145, 296 (1982)]. Many of these strains appear to lack the toxin gene, as measured by hybridization with a radioactive DNA probe [Kaper, J.B. et al. Infect. Immun. 32, 661 (1981)].

### 2. Mutagenized Attenuated Strains

Classical Inaba 569B has been mutagenized with nitrosoguanide (NTG) and hypotoxinogenic mutant isolated [Finkelstien, R.A. et al. J. Infect. Dis. 129, 117 (1974); Holmes, R.K. et al. J. Clin. Invest. 55, 551 (1975). This mutant strain, M13, was fed to volunteers. Diarrhea did not occur but the strain colonized poorly. Challenge studies demonstrated that some protective efficacy was conferred by immunization with multiple doses [Woodward, E. et al. Develop. Biol. Stand. 33, 108, (1976)].

El Tor Ogawa 3083 has also been mutagenized [Honda, T. et al. Proc. Nat. Acad. Sci. 76, 2052 (1979)]. Brute force selection and analysis of thousands of colonies yielded one isolate that continued to produce the immunogenic B subunit while failing to produce detectable A subunit or holotoxin. The one isolate, Texas Star-SR, fulfilled these criteria. Texas Star-SR produces normal or increased amount of B subunit but is negative in assays for holotoxin activity or A subunit activity.

Texas Star-SR has been extensively evaluated in volunteers (see, e.g., Levine M.M. et al. Acute Enteric, supra (1981)). Groups of five volunteers received two 10⁹ organism doses one week apart and eighteen more volunteers ingested two 2 x 10¹⁰ organism doses one week apart. Some degree of diarrhea was seen in sixteen of the sixty-eight vaccines (24%). In only one individual did the total stool volume exceed 1.0 liter (1464 ml). Typically, the vaccine-induced diarrhea consisted of two or three small, loose stools totalling less than 400 ml in volume. Vaccine organisms were recovered from coprocultures of approximately one-half of the vaccine recipients. Where jejunal fluid was cultured (recipients of doses of 10⁸ or more vaccine organisms), cultures were positive in thirty-five of forty-six vaccines (76%). Hundreds of Texas Star clones recovered from coprocultures and jejunal fluid cultures were examined for cholera holotoxin by the sensitive Y-1 adrenal cell assay; none were positive.

Significant rises in serum antitoxin were detected in only 29% of the vaccines; however, 93% manifested significant rises in serum vibriocidal antibody and the titers were substantially close to those encountered following infection with pathogenic V. cholerae. In experimental challenge studies in volunteers, Texas Star-SR was found to confer significant protection against challenge with both EL Tor Ogawa And El Tor Inaba vibrios. One or two doses of Texas Star-SR attenuated oral vaccine confers good protection against El Tor cholera.

It is clear that the use of attenuated strains has intrinsic advantages since such strains mimic infection-derived immunity to cholera. However, the Texas Star-SR strains suffers from certain drawbacks. To begin with, mutagenesis (e.g., with nitrosoguanidine) induces multiple mutations, not all of which are necessarily recognized. Furthermore, the precise genetic lesion that is presumed to be responsible for the attenuation of Texas Star-SR is not known. In addition, Texas Star-SR may revert to virulence, like any pathogen mutated with nitrosoguanidine.

Applicants of the present invention have isolated, by novel method, deletion mutants of a virulent strain of Vibrio cholerae known to produce both immunity and disease in volunteers. The deletions are restriction endonuclease fragments. The vaccine strains of the present invention have been specifically altered through the use of recombinant DNA techniques to render the avirulent without affecting other components necessary for immunity. This attenuation was accomplished by using restriction endonucleases which cleave the DNA of the bacterium at specific sites, to specifically delete the genes responsible for cholera toxin (i.e., the ctx gene). Plasmids carrying the ctx gene were digested with restriction endonucleases to delete the ctx gene, but were constructed to retain extensive lengths of flanking DNA of the V. cholerae chromosome. Conjugal gene transfer of the plasmids into V. cholerae yielded an avirulent V. cholerae strain carrying the extrachromosomal copies of the plasmids. Subsequent conjugation with cells having other plasmids produced, after appropriate selection of selectable plasmid markers, V. cholerae strains having deletions in the ctx regions. Such nontoxigenic deletion mutants would then be capable of colonizing the small intestine and stimulating local, protective immunity directed against the bacterial cell. After the transient colonization episode, the vaccine would be protective against subsequent infection with virulent toxigenic V. cholerae strains.

The genes for V. cholerae toxin have been cloned [Pearson, G.D.N. et al. Prod. Nat. Acad. Sci. 79, 2976 (1982); Kaper, J.B. et al. Amer. Soc. Micribiol. Abstr. Annu. Meeting, Atlanta, Georgia, 36 (1982); Kaper, J.B. et al. Symposium on Enteric Infections in Man and in Animals: Standardization of Immunological Procedures, Dublin, Ireland, Abstract No. 2.5 (1982)]. Toxin structural gene deletion mutants of V. cholerae have been isolated, but only by infection with mutagenic vibriophages capable of integration at random sites along to chromosome [Mekalanos, J.J. et al. Proc. Nat. Acad. Sci. 79, 151, (1982)]. Recombination in Vibrio cholerae has been reported, but it has not been used to isolate restriction fragment deletions in the ctx genes for vaccination purposes [Parker, C. et al. J. Bact. 112, 707 (1972); Johnson, S.R. et al. Molec. Gen. Genet. 170, 93 (1979); Sublett, R.D. et al. Infect. Immun. 32 1132 (1981) and Thomson, J.A. et al. J. Bact. 148, 374 (1981)].

### BRIEF DESCRIPTION OF THE INVENTION

A culture of Vibrio cholerae is described comprising a Vibrio cholerae strain having a restriction endonuclease fragment of DNA deleted to confer avirulence and to retain capacity to colonize the intestine of a host animal. The DNA fragment deleted may code for the V. cholerae toxin or portions thereof such as the A₁ subunit. One isolated deletion mutant encompasses a deletion in the ctx gene, as defined by Acc I restriction endonuclease sites.

A method of isolating such deletion mutants of Vibrio cholerae is also described, comprising the steps of
(a) constructing a first plasmid comprising Vibrio cholerae flanking sequences of one or more deleted restriction endonuclease fragments and a gene for a first selectable marker of foreign origin ligated to said flanking sequences to substitute for and to be in the place of said deleted fragment, wherein said sequences are of sufficient length to promote detectable in vivo recombination;
(b) mating a virulent strain of Vibrio cholerae with a first microorganism carrying the first plasmid;
(c) selecting for Vibrio cholerae expressing the first selectable marker;
(d) mating the selected product of step (c) with a second microorganism carrying a second plasmid with a second selectable marker, said second plasmid being incompatible with the first plasmid; and
(e) selecting for Vibrio cholerae expressing both the first selectable marker and the second selectable marker.

A second culture of Vibrio cholerae is described comprising a Vibrio cholerae strain having a first restriction endonuclease fragment of DNA deleted to confer avirulence and retain capacity to colonize the intestine of a host animal and having a second restriction endonuclease fragment of DNA coding for zonula occludens toxin (ZOT) deleted to reduce residual diarrhea in the host animal. The first DNA fragment deleted may code for the V. cholerae toxin or portions thereof such as the A₁ subunit. One isolated deletion mutant encompasses a deletion in the ctx gene, as defined by Acc I restriction endonuclease sites, and a deletion in the zot gene. Another isolated deletion mutant encompasses a deletion in the ctx gene, as defined by Xba I and Cla I restriction endonuclease sites, and a deletion in the zot gene, as defined by Stu I and Acc I restriction endonuclease sites

A method of isolating such deletion mutants of Vibrio cholerae is also described, comprising the steps of
(a) constructing a first plasmid comprising Vibrio cholerae flanking sequences of one or more deleted restriction endonuclease fragments and a gene for a selectable marker of foreign origin ligated to said flanking sequences to substitute for and to be in place of said deleted fragment, wherein said sequences are of sufficient length to promote detectable in vivo recombination;
(b) mating a virulent strain of Vibrio cholerae with a first microorganism carrying the first plasmid;
(c) selecting for Vibrio cholerae expressing the first selectable marker;
(d) mating the selected product of step (c) with a second microorganism carrying a second plasmid with a second selectable marker, said second plasmid being incompatible with the first plasmid;
(e) selecting for Vibrio cholerae expressing both the first selectable marker and the second selectable marker;
(f) constructing a third plasmid comprising Vibrio cholerae flanking sequences of one or more deleted restriction endonuclease fragments homologous to those described in step (a) but differing in the absence of a selectable marker of foreign origin;
(g) mating the selected product of step (e) with a third microorganism carrying a third plasmid described in step (f); and
(h) selecting for Vibrio cholerae which no longer expresses the first selectable marker.

This method may be used for ZOT minus only strains or for making a ZOT minus derivative of a strain which is already deleted for cholera toxin genes.

A third culture of Vibrio cholerae is described, comprising a Vibrio cholerae strain having a region of the chromosomal DNA coding for cholera toxin and zonula occludens toxin (ZOT) deleted. A method of isolating such deletion mutants of Vibrio cholerae is also described comprising the steps of
(a) constructing a plasmid comprising Vibrio cholerae sequences coding for cholera toxin and zonula occludens toxin and a gene for a selectable marker of foreign origin, wherein said plasmid is incapable of replicating extrachromosomally in Vibrio cholerae ;
(b) mating a microorganism carrying said plasmid with a virulent strain of Vibrio cholerae containing said sequences inserted between flanking identical copies of a second sequence such as RS1 elements of sufficient length to promote detectable in vivo recombination;
(c) selecting for Vibrio cholerae expressing said selectable marker;
(d) growing the selected product of (c) in the absence of the selective agent;
(e) selecting for Vibrio cholerae which no longer express the selective marker, and therefore have a region of the chromosomal DNA coding for cholera toxin and zonula occludens toxin deleted.

A fourth culture of Vibrio cholerae is described, comprising a Vibrio cholerae strain having a region of chromosomal DNA coding for cholera toxin and zonula occludens toxin deleted, and having inserted a mercury resistance gene and DNA coding for B subunit of Vibrio cholerae toxin. A method of isolating such deletion mutants is also described comprising the steps of:
(a) constructing a plasmid comprising Vibrio cholerae sequences coding for cholera toxin and zonula occludens toxin and a gene for a selectable marker of foreign origin, wherein said plasmid is incapable of replicating extrachromosomally in Vibrio cholerae;
(b) mating a microorganism carrying said plasmid with a virulent strain of Vibrio cholerae containing said sequences coding for cholera toxin and zonula occludens toxin inserted between flanking identical copies of a second sequence of sufficient length to promote detectable in vivo recombination;
(c) selecting for Vibrio cholerae expressing said selectable marker;
(d) growing the selected product of (c) in the absence of the selective agent;
(e) selecting for Vibrio cholerae which no longer express the selective marker, and therefore have a region of the chromosomal DNA coding for cholera toxin and zonula occludens toxin deleted;
(f) constructing a second plasmid comprising a mercury resistance gene and DNA coding for B subunit of Vibrio cholerae toxin and a gene for a second selectable marker of foreign origin wherein said plasmid is incapable of replicating extrachromosomally in Vibrio cholerae, and wherein sequences of sufficient length to promote detectable in vivo recombination flank said mercury resistance gene and DNA coding for B subunit of Vibrio cholerae toxin;
(g) mating a microorganism carrying said second plasmid with said Vibrio cholerae recited in step (e) containing sequences homologous to said sequences of sufficient length to promote detectable in vivo recombination;
(h) selecting for Vibrio cholerae expressing said second selectable marker;
(i) growing the selected product of step (h) in the absence of the second selective agent;
(j) selecting for Vibrio cholerae which no longer express the second selective marker; and
(k) screening said Vibrio cholerae recited in step (j) for Vibrio cholerae that have a mercury resistance gene and DNA coding for B subunit of Vibrio cholerae toxin and have a region of chromosomal DNA coding for cholera toxin and zonula occludens toxin deleted.

The Vibrio cholerae deletion mutants of this invention are useful in vaccination against cholera.

One Vibrio cholerae strain of the present invention, designated CVD101, is expected to confer substantially close to 100% efficacy in humans against subsequent infection with a strain of a similar serotype. Other Vibrio cholerae strains of the present invention, designated by the second culture, and the third culture such as CVD109, are expected to confer substantial protection in humans against subsequent infection with a strain of a similar serotype and to avoid undesirable side effects such as diarrhea and nausea, and cramping. Another Vibrio cholerae strain of the present invention, CVD110, is designated by the fourth culture.

### BRIEF DESCRIPTION OF THE DRAWINGS

Figure 1. V. cholerae N16961 (pJBK55) (Ap^{r})

Figure 2. Processes of crossing-over and conjugal gene transfer to construct V. cholerae JBK56.

Figure 3. V. cholerae JBK56.

Figure 4. Scheme for construction of JBK21.

Figure 5. Scheme for construction of pJBK54.

Figure 6. Scheme for construction of V. cholerae JBK56.

Figure 7. Recombination in vivo by cross over and elimination of ctx gene.

Figure 8. Scheme for construction of pJBK51.

Figure 9. Scheme for construction of pCVD14 and pCVD15.

Figure 10. Scheme for construction of pJBK108.

Figure 11. Scheme for construction of pJBK107.

Figure 12. DNA sequence of (top) the Xba I and Cla I sites, which determine the ends of the deleted Xba I-Cla I 550bp fragment of the A subunit in Ogawa 395, and for (bottom) the junction in CVD101 after deletion of this fragment and insertion of an Xba I linker.

Figures 13A and 13B. Effect of V. cholerae culture supernatant on ileal short circuit current (Isc) and tissue ionic conductance (Gt). Values are means for 6 animals at each time-point; brackets are 1 standard error a, Effect of V. cholerae 395 supernatants on Isc (solid lines) and Gt (dashed lines). b, Effect of V. cholerae 395 (solid Line), CVD101 (long dashed line) and 395N1 (dotted line) supernatants on Gt. Medium control (short dashed line) consisted of uninoculated culture medium.

Figures 14A, 14B, 14C, and 14D. Wheat germ agglutinin - horseradish peroxidase (WGA-HRP) permeability assay on rabbit ileal tissues exposed to culture supernatants of various V. cholerae strains. a, medium control; b, V. cholerae 395; c, V. cholerae 395N1; d, V. cholerae CVD101.

Figures 15A, 15B, and 15C. Freeze-fracture studies of rabbit ileal tissue exposed to culture supernatants of V. cholerae a, An intact ZO with numerous intersections (arrowheads) between junctional strands M, microvilli. b, An affected ZO from ileal tissue exposed to V. cholerae 395; the reticulum appears simplified due to greatly decreased incidence of strand intersections. c, Quantitation of ZO complexity in tissues exposed to culture supernatants or broth control.

Figure 16. Reversibility of Gt variations induced by V. cholerae 395 supernatant. Culture supernatants of V. cholerae (triangles) and uninoculated medium (squares) were added and removed at the time indicated by arrows.

Figure 17. Scheme for construction of CVD109. The zot and ctx genes are adjacent to each other on the V. cholerae chromosome and are in a region of the chromosome which contains multiple copies of a 2700 sequence sequence called RS1 (repetitive sequence). RS1 elements are on both sides of zot and ctx genes in virulent V. cholerae strain E7946 (El Tor biotype, Ogawa serotype). The zot and ctx genes are shown by a large open or hash-marked arrow. RS1 sequences are shown by a smaller, solid arrow.

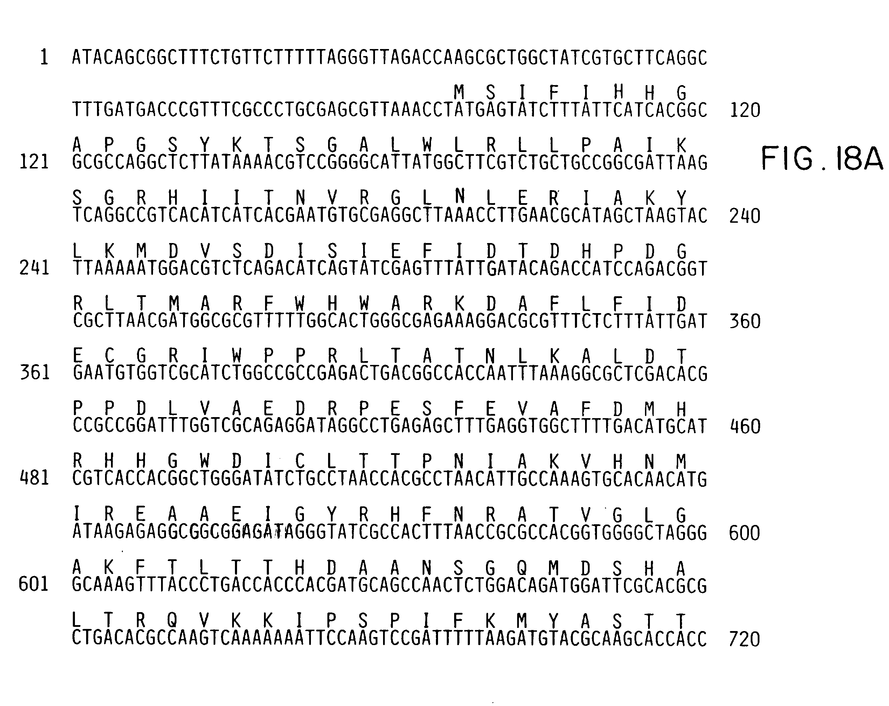

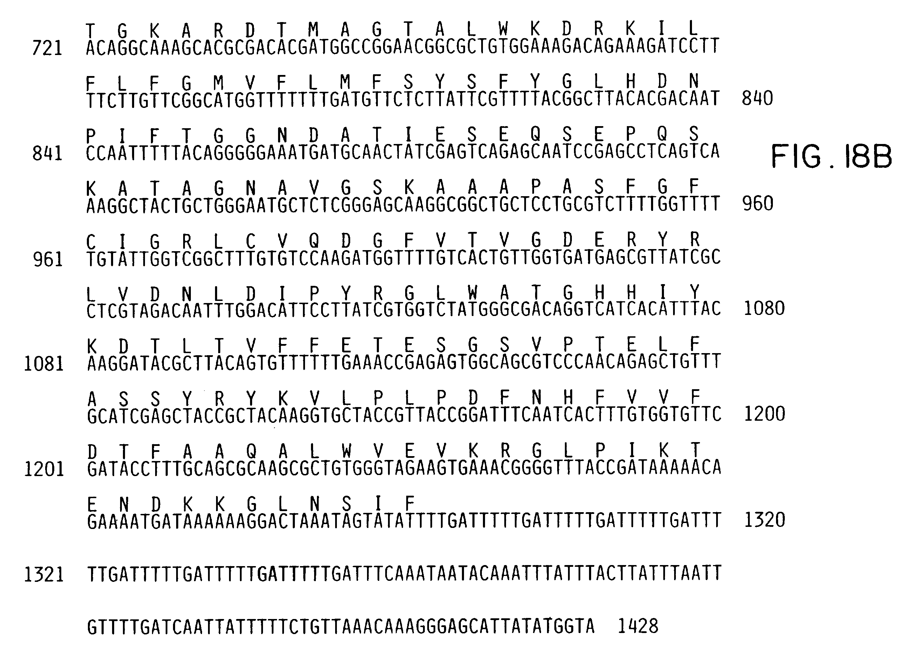

Figure 18 (pages 1 and 2). DNA sequence of the zot gene for zonula occludens toxin from nucleotides number 1 to 1428. Letters above the DNA sequence indicate the predicted amino acid sequence of the ZOT protein encoded by the zot gene.

Figure 19. Scheme for construction of plasmid pCVD621 and plasmid pCVD622.2B.

Figure 20. Scheme for construction of CVD110.

Figure 21 (pages 1 to 7). DNA sequence of the ctxB gene and mer gene inserted into the HlyA gene.

Abbreviations for restriction endonuclease sites in the drawings are as follows:
A = Acc I restriction endonuclease site
B = Bgl II restriction endonuclease site
C = Cla I restriction endonuclease site
E = Eco RI restriction endonuclease site
H = Hind III restriction endonuclease site
P = Pst I restriction endonuclease site
S = Sal I restriction endonuclease site
X = Xba I restriction endonuclease site
K = Kpn I restriction endonuclease site

Other abbreviations in the drawings and elsewhere include:
Ap = Ampicillin resistance gene
Ap^{r} = Ampicillin resistance phenotype
Ap^{s} = Ampicillin sensitive phenotype
Chrom = Chromosome
Cm = Chloramphenicol resistance gene
CT = Cholera toxin
ctx = gene for cholera toxin
CTA = gene for A subunit of cholera toxin
ctxA = gene for A subunit of cholera toxin
CTB = gene for B subunit of cholera toxin
ctxB = gene for B subunit of cholera toxin
hylA = gene for hemolysin
kb = Kilobases
mer = gene for mercury resistance
p = plasmid
Su = Sulfonamide
Su^{r} = Sulfonamide resistance phenotype
Tc = tetracycline
Tc^{s} = tetracycline sensitive phenotype
Tp = Trimethoprin
zot = gene for zonula occludens toxin

### DETAILED DESCRIPTION OF THE INVENTION

The principle of the present invention is the isolation of a Vibrio cholerae vaccine strain specifically altered through recombinant DNA technology to render it avirulent without affecting other components necessary for immunity. This attenuation was accomplished by restriction endonuclease digestion of plasmids carrying appropriate V. cholera sequences, to specifically delete the genes coding for cholera toxin, or portion thereof. Conjugal gene transfer of these digested plasmids, followed by procedures for selecting in vivo recombinants with virulent host V. cholera, resulted in strains without the toxin genes portion thereof. It will be understood that the methods of the present invention are applicable to the isolation of other deletion mutants of virulent V. cholerae, or to the isolation of strains having all or part of such deleted sequences reintroduced into the V. cholerae cell.

The starting material for the vaccine was the toxigenic Vibrio cholerae strain N16961, which has been demonstrated to produce in volunteers both typical diarrheal disease and strong, protective immunity to subsequent infection [Levine, M.M. et al., Acute enteric, supra. 1981]. The region of the bacterial chromosome which was found to be responsible for production of cholera toxin was cloned into the plasmid cloning vehicle pBR325, after screening Hind III digest of V. cholerae with an E. coli heat-labile enterotoxin gene probe [Kaper et al. Amer. Soc., supra; Kaper et al. Symposium, supra]. The V. cholerae chromosome fragment was found to contain all genes necessary for toxin production. Next, this chromosomal region was then analyzed and mapped for the exact portions containing the toxin genes [Kaper, J.B. et al. Lancet II, 1162 (1981)]. Restriction enzymes were employed to cut out the DNA fragments containing these genes and a DNA fragment encoding a selectable marker (e.g., resistance to ampicillin) was inserted by ligation. The ampicillin resistance gene and the flanking Vibrio DNA were then cloned in a derivative of pRK290 which can be transferred from E. coli to V. cholerae. The resulting plasmid, pJBK55, was transferred from E. coli t K-12 to V. cholerae N16961 by conjugation.

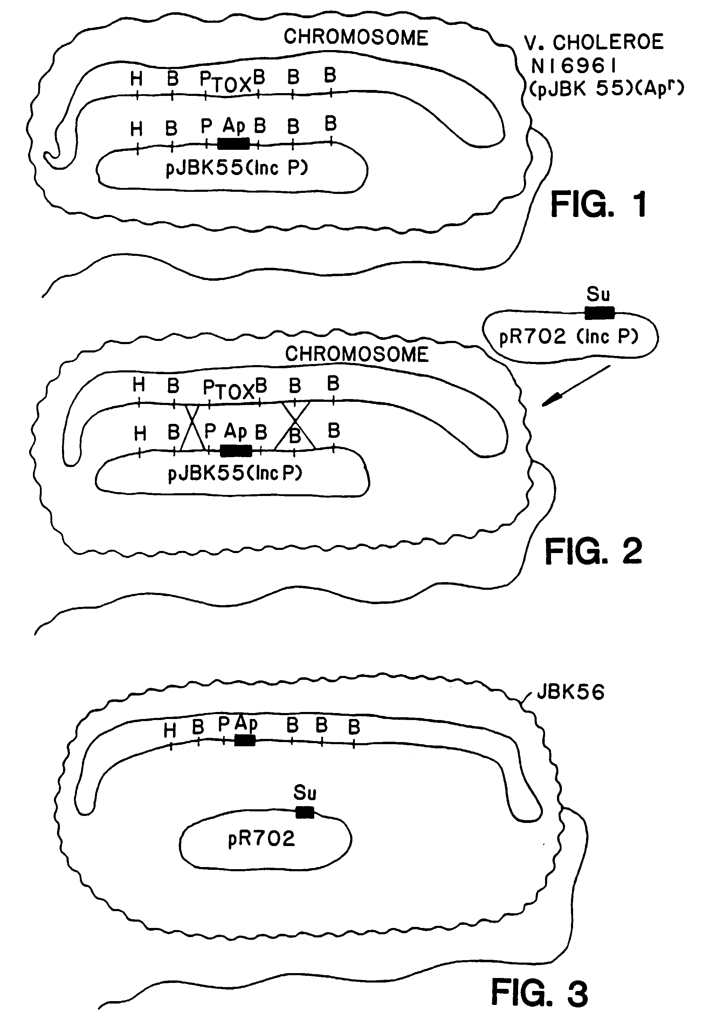

The resulting strain, V. cholerae N16961 (pJBK55) (Ap^{r}) contained a region in its chromosome having intact toxin genes and, in an extrachromosomal state, a plasmid containing this same region with the toxin genes deleted and a gene for ampicillin substituted. (See Figure 1.) At a low frequency, perhaps one in 10⁶ to one in 10⁸, the identical regions flanking the chromosomal toxin genes and the extrachromosomal (plasmid) ampicillin resistance gene will exchange and "cross over" or undergo in vivo recombination so that the region of DNA containing the resistance gene displaces the toxin gene on the chromosome (Figure 2). This rare event is selected by testing a mixture of mutated and non-mutated cells for individual cells which are able to serve as host for an incoming incompatible plasmid [Ruvkun, G.B. et al. Nature 289, 85 (1981)]. Plasmids are divided into groups designated A through W, the members of which cannot stably coexist with each other. For example, a plasmid of incompatibility group P cannot be stably maintained in the same cell as another P group (Inc P) plasmid. Thus, Inc P plasmids, such as R702, which specify resistance to sulfonamide, cannot be maintained in a cell which has another Inc P. plasmid such as PRK 290, pJBK45, or pJBK55. Therefore, R702 can be maintained in a strain in which the ampicillin resistance has recombined into the chromosome but not one in which an Inc P Plasmid (e.g. pJBK55) is replicating extrachromosomally. By mating an E. coli strain containing Inc P R702 (sulfonamide resistant) and V. cholerae pJBK55 (ampicillin resistant) and selecting for V. cholerae which are resistant to both ampicillin and sulfonamide, colonies are isolated in which the sulfonamide resistance is mediated extrachromosomally by p702 and the ampicillin resistance is mediated chromosomally through substitution of the ampicillin resistance gene for the toxin gene (Figure 3). One such strain, designated V. cholerae JBK56 was isolated and when tested for toxin production was found to be nontoxinogenic.

The final version of the vaccine strain, JBK70, was produced by substituting resistance to ampicillin, a therapeutically useful antibiotic, with resistance to mercury. This substitution was accomplished by cloning a gene for mercury resistance directly into the ampicillin resistant gene of pJBK55, thereby inactivating ampicillin resistance and conferring mercury resistance. The resulting plasmid, pJBK66 was also incompatible with R702 and was transferred to V. cholerae JBK56. A mutant in which the mercury resistance was recombined into the chromosome was selected using the Inc P plasmid R702 and selecting for V. cholerae which were ampicillin sensitive, mercury resistant, and sulfonamide resistant. A spontaneous derivative was later selected which was cured of pR702. The final mutant, JBK70, was nontoxinogenic and resistant to mercury only.

The vaccine strain V. cholerae JBK70 is one of the Inaba serotype. The other major serotype of V. cholerae is the Ogawa serotype. It is expected that a vaccine prepared from one serotype will protect against the other serotype (34). In the event that this is not the case, a live vaccine strain can be prepared from an Ogawa serotype and protection in volunteers [Levine, M.M. et al. Acute enteric, supra (1981)]. The exact mutation created in strain V. cholerae Inaba JBK56 was recreated in strain E7946 by directly transferring the region of the chromosome containing the ampicillin resistance in place of the toxin gene in JBK56 into E7946 through genetic recombination mediated by P, the sex factor of V. cholerae [Parker, C. et al., supra]. The P factor, which is distinct from Inc P plasmid, was transferred into JBK56 and was then mated with a rifampin resistant mutant of E7946. By selection of a mutant which was resistant to both ampicillin and rifampin, a vaccine strain was isolated which was of the Ogawa serotype with the toxin genes completely deleted.

If antibacterial immunity is insufficient for protection, then an antitoxic component can be added by adding back the genes for production of cholera toxin B but not A subunit. This has been accomplished by cloning the B subunit gene into the cloning vector pMS9. The resulting plasmid, pJBK51, produces high levels of B subunit and was reintroduced into the nontoxic vaccine strain V. cholerae JBK70 to make an attenuated vaccine strain JBK70 ([JBK51) which fails to produce the A subunit.

The vaccine strains of the present invention are derived inter alia from V. cholerae N16961 having the serotype Inaba. It will be understood that other strains or other biotypes and serotypes can be used to substitute for N16961 to produce vaccine strains having specific deletions in the ctx gene or genes, or in other locations along the V. cholerae chromosome. Since the object of isolating such vaccine strains is to mimic the infection process without associated pathological phenomena, site-directed mutagenesis of virulent strains, as described in this application, produces substantial possibilities in the prophylactic vaccination against cholera.

For example, applicants have produced another V. cholerae vaccine strain CVD101, characterized by a deletion of most of the A subunit gene in 2 copies of the ctx gene. It is expected that the efficacy of CVD101 is substantially close to 100%, since the parent strain 395 confers 100% efficacy.

Construction of CVD101 followed in general the principles outlined supra, e.g. the construction of JBK70, except that the resulting CVD101 had no resistance gene that needed curing. The final step in isolating the second and find in vivo recombinant included a scheme for selecting sensitivity to an antibiotic e.g. tetracycline sensitivity, whereas the parent strain had inserted at the location of the A gene of CT a tetracycline resistance gene. It will be understood that such antibiotic sensitivity is another example of a selectable marker.

Production of vaccine strains can be performed by a variety of methods, including the following: Vibrio cholerae is subcultured from stock cultures into brain/-heart infusion agar (BHIA) and grown at 37°C overnight. Identity is tested with group-and type-specific antisera and twenty to thirty colonies are suspended in BHI broth. Preincubated BHIA plates are inoculated with BHI suspension. After incubation for five to six hours, each plate is harvested with 5 ml of sterile saline buffered to pH 7.2 ± 0.1. Harvested organisms are centrifuged in the cold at 750 g for ten minutes, resuspended and washed twice in four-times the original volume. The suspension is standardized spectrophotometrically and diluted to approximate the number of organisms required for vaccination (ca 10⁶, which varies depending on the results of volunteer studies). Replicate, pour-plate quantitative cultures are made of the inocula before and after challenge to confirm inoculum size. The final inoculum is examined with Gram's stain and agglutinated with homologous antiserum prior to feeding.

The Vibrio cholerae strains of the present invention can be administered by the oral route. Two grams of NaHCO₃ are dissolved in five ounces of distilled water. Volunteers drink four ounces of the NaHCO₃/water; one minute later the volunteers ingest the vibrios suspended in the remaining one ounce of NaHCO₃/water. Volunteers are NPO ninety minutes pre- and postinoculation.

With regard to safety, the major concern is that the vaccine strain does not revert to toxigencity (i.e., produce intact cholera toxin) which could cause disease. The two major assays for testing toxin are the Y-1 adrenal cell assay [Sack, D.A. et al. Infect. Immjun. 11, 334 (1975)] and the enzyme-linked immunosorbent assay (ELISA) [Sack, D.A. et al. J. Clin. Micro. 11, 35 (1980)]. The vaccine strain (JBK70) has been repeatedly tested in these two assays and found to be negative each time. Far more important, however, are the genetic assays performed for the presence of toxin genes. The DNA for cholera toxin genes can be radioactively labeled and used as a specific probe to identify other cholera toxin genes in the strain, according to the method of Southern, E.M. J. Mol. Bio. 98, 503 (1975). When tested by this method, the vaccine strain described in the invention possesses no detectable genetic material that can enclose cholera toxin. The vaccine has also been tested in an infant mouse model, according to Baselski, V. et al. Infect. Immun. 15, 704 (1977). After repeated (ten in all) serial passages, no fluid accumulation (i.e., evidence of disease has been found. As expected, JBK70 was found to colonize the infant mouse intestine.

In order to avoid undesirable side effects of the vaccine strains, such as diarrhea and nausea, cramping, and other symptoms, the vaccine strains may further comprise a second restriction endonuclease fragment of DNA coding for zonula occludens toxin (ZOT) deleted.

A culture of Vibrio cholerae comprises a Vibrio cholerae strain having a first restriction endonuclease fragment of DNA deleted to confer avirulence and retain capacity to colonize the intestine of a host animal and having a second restriction endonuclease fragment of DNA coding for zonula occludens toxin (ZOT) deleted to reduce residual diarrhea in the host animal. The first DNA fragment deleted may code for the V. cholerae toxin or portions thereof such as the A₁ subunit. One isolated deletion mutant encompasses a deletion in the ctx gene, as defined by Acc I restriction endonuclease sites, and a deletion in the zot gene. Another isolated deletion mutant encompasses a deletion in the ctx gene, as defined by Xba I and Cla I restriction endonuclease sites, and a deletion in the zot gene, as defined by Stu I and Acc I restriction endonuclease sites

A method of isolating such deletion mutants of Vibrio cholerae comprises the steps of
(a) constructing a first plasmid comprising Vibrio cholerae flanking sequences of one or more deleted restriction endonuclease fragments and a gene for a selectable marker of foreign origin ligated to said flanking sequences to substitute for and to be in place of said deleted fragment, wherein said sequences are of sufficient length to promote detectable in vivo recombination;
(b) mating a virulent strain of Vibrio cholerae with a first microorganism carrying the first plasmid;
(c) selecting for Vibrio cholerae expressing the first selectable marker;
(d) mating the selected product of step (c) with a second microorganism carrying a second plasmid with a second selectable marker, said second plasmid being incompatible with the first plasmid;
(e) selecting for Vibrio cholerae expressing both the first selectable marker and the second selectable marker;
(f) constructing a third plasmid comprising Vibrio cholerae flanking sequences of one or more deleted restriction endonuclease fragments homologous to those described in step (a) but differing in the absence of a selectable marker of foreign origin;
(g) mating the selected product of step (e) with a third microorganism carrying a third plasmid described in step (f); and
(h) selecting for Vibrio cholerae which no longer expresses the first selectable marker.

This method may be used for ZOT minus only strains or for making a ZOT minus derivative of a strain which is already deleted for cholera toxin genes.

Another culture of Vibrio cholerae comprises a Vibrio cholerae strain having a region of the chromosomal DNA coding for cholera toxin and zonula occludens toxin (ZOT) deleted. A method of isolating such deletion mutants of Vibrio cholerae comprises the steps of
(a) constructing a plasmid comprising Vibrio cholerae sequences coding for cholera toxin and zonula occludens toxin and a gene for a selectable marker of foreign origin, wherein said plasmid is incapable of replicating extrachromosomally in Vibrio cholerae ;
(b) mating a microorganism carrying said plasmid with a virulent strain of Vibrio cholerae containing said sequences to promote detectable in vivo recombination;
(c) selecting for Vibrio cholerae expressing said selectable marker;
(d) growing the selected product of (c) in the absence of the selective agent;
(e) selecting for Vibrio cholerae which no longer express the selective marker; and therefore have a region of the chromosomal DNA coding for cholera toxin and zonula occludens toxin deleted. Step (b) may comprise: (b) mating a microorganism carrying said plasmid with a virulent strain of Vibrio cholerae containing said sequences inserted between flanking identical copies of a second sequence such as RS1 elements of sufficient length to promote detectable in vivo recombination.

The Vibrio cholerae deletion mutants of this invention are useful in vaccination against cholera.

Herein reported is a new toxic factor elaborated by V. cholerae which increases the permeability of the small mucosa by affecting the structure of the intercellular tight junctions or zonula occludens (ZO) (the paracellular pathway of ion transport). Production of this factor by V. cholerae correlates with diarrheagenicity in volunteers. By disturbing the normal absorptive processes of the small intestine via the paracellular pathway, this factor could be responsible for the residual diarrhea induced by ctx deletion mutants of V. cholerae and may contribute to the severe diarrhea that distinguishes cholera from other diarrheal diseases.

Changes in intestinal function induced by three strains of V. cholerae, one wild type and two attenuated vaccine strains, were examined. V. cholerae strain 395, classical biotype, Ogawa serotype, is a highly virulent strain which has been extensively characterized in volunteer studies conducted at the Center for Vaccine Development. This strain induces diarrhea with a mean stool volume of 5.5 liters (range of 0.3 to 44 1) in greater than 90% of volunteers ingesting 10⁶ organisms [Levine, M.M. et al, Infect. Immun. 56, 161-167 (1988)]; [Levine, M.M., Cholera and Related Diarrheas, 195-203] (Karger, Basel, 1980). Cholera diarrhea is principally due to the enzymatic effects of the A subunit of CT on intestinal mucosa. The CT A subunit, encoded by ctx, stimulates adenylate cyclase and results in net secretion of fluid into the intestinal lumen. Gill, D.M. Adv. Cyclic Nucleotide res. 8, 85-118 (1977). V. cholerae vaccine strain CVD101 is a ctx deletion mutant of 395 in which 94% of the sequences encoding the A₁ peptide of CT have been removed. Surprisingly, although CVD101 no longer produces active CT, this strain caused mild to moderate diarrhea (mean stool volume of 0.9 l with a range of 0.3 to 2.1 l) in 54% of volunteers ingesting this organism. A second derivative of 395, vaccine strain 395N1, constructed by Mekalanos, et al.,Nature 306, 551-557 (1983), lacks ca. 77% of the sequences encoding the A₁ peptide by applicants' calculation. In contrast to CVD101, 395N1 induced very mild diarrhea (0.3 l stool volume) in only 1 of 21 volunteers (P=0.002 compared to 13 of 24 volunteers with diarrhea after ingestion of CVD101). [Herrington, D.A. et al. J. Exp. Med. 168, 1487-1492 (1982)]. Since these strains were similar in their ability to colonize the intestine, applicants hypothesize that CVD101 produces a secretogenic factor which is expressed weakly or not at all by 395N1 and that this factor is responsible for the diarrhea seen in volunteers ingesting CVD101.

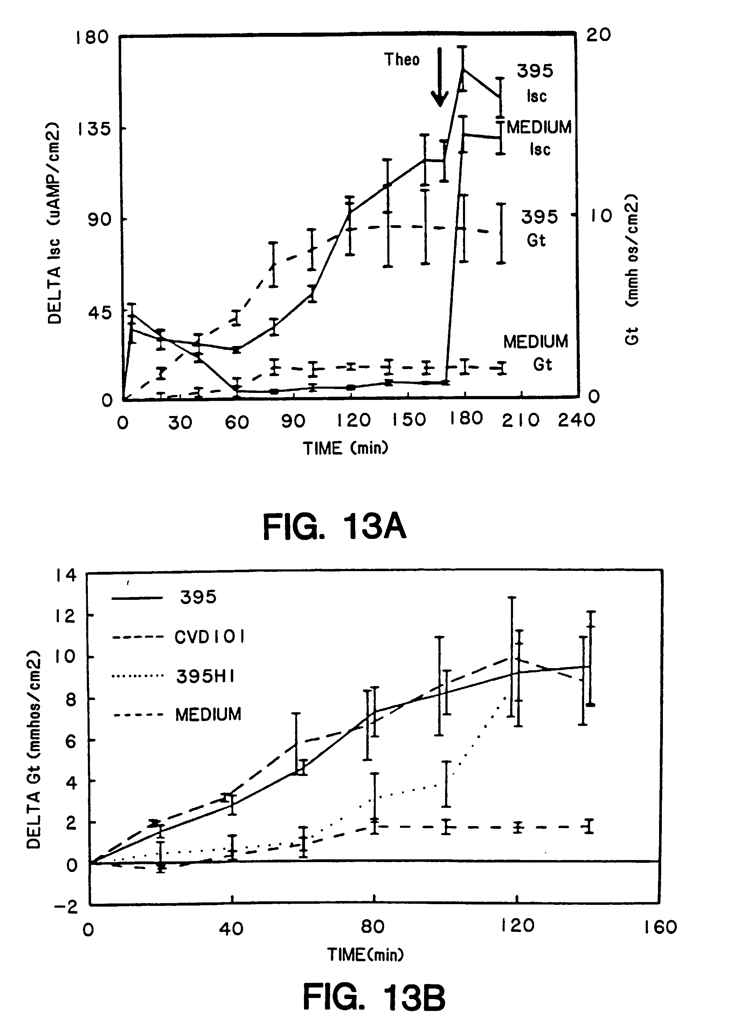

These strains were studied using rabbit intestinal tissue mounted in Ussing chambers, a classic technique for studying the transport process across intestinal tissue. Supernatants of V. cholerae cultures were added to the chambers and potential difference (PD) and short circuit current (Isc) were measured. PD is the difference in voltage measured on the mucosal side vs. the serosal side of the tissue and Isc is the amount of current needed to nullify the PD. From these measurements, tissue conductance (Gt) was calculated using Ohm's law: Isc = PD x Gt. Applicants first studied the effect of supernatants of the wild type strain 395 on these parameters using uninoculated culture media added to matched ileal tissue from the same animal as a negative control. Fig. 13A shows the Isc and Gt variations obtained. The initial peaks in Isc and PD that occurred in both negative controls and test samples were most likely due to the cotransport of Na and nutrients present in the media. In the negative control, Isc and PD returned to baseline values after approximately one hour and subsequently Isc, PD and Gt remained unchanged for the rest of the experiment. In contrast, tissues exposed to strain 395 supernatant exhibited a significant increase in Gt, reaching a maximum value after 2 hrs of incubation. In such samples, the Isc never returned to the baseline, but a steady state period for Isc was noted between 40 and 60 minutes. Since Isc is equivalent to PD x Gt and the observed PD after 60 min. was similar to the initial value (data not shown), the significant increase in Isc in 395-treated tissues at that time point can only be due to an increase in Gt (see Fig. 13A time 60 Min.) (12). After 60 min., Isc began to rise again along with PD in 395-treated tissues. This second phase probably reflects the effect of cholera toxin on ion fluxes since purified CT increases Isc in rabbit ileal tissue only after a lag time of at least 40 minutes. These data suggest that there are two factors expressed by V. cholerae 395 that can alter ion transport in Ussing chambers. One factor, cholera toxin, induces an increase in Isc and PD beginning ca. 60 minutes after addition of culture supernatant while a second factor induces an immediate increase in tissue conductance which is observable within 20 minutes after addition of culture supernatant.

Gt variation induced by culture supernatants of the attenuated V. cholerae strains CVD101 and 395N1 was next studied. CVD101 induced an immediate increase in Gt which was indistinguishable from that seen with 395 (Fig. 13B). In contrast, 395N1 induced no immediate increase in Gt; Gt variation in 395N1-treated tissues was similar to the negative broth control and significantly lower than that seen with 395 and CVD101 for almost 100 min of incubation. After this period, Gt modification in tissues exposed to 395, CVD101 and 395N1 were similar. These results suggest that 395N1 produces lower amounts or a less active form of the factor responsible for this increase in Gt.

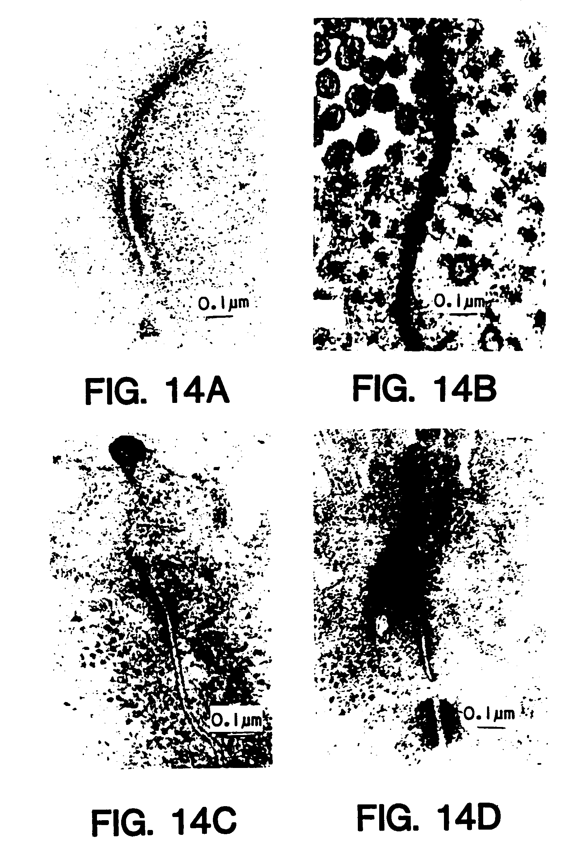

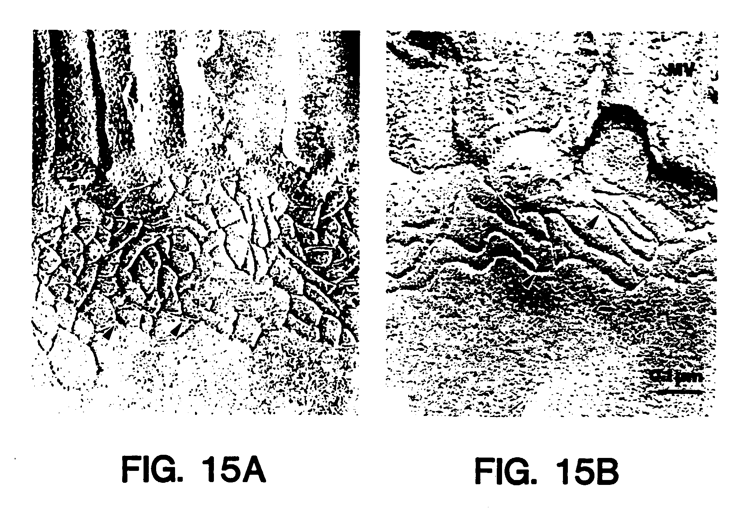

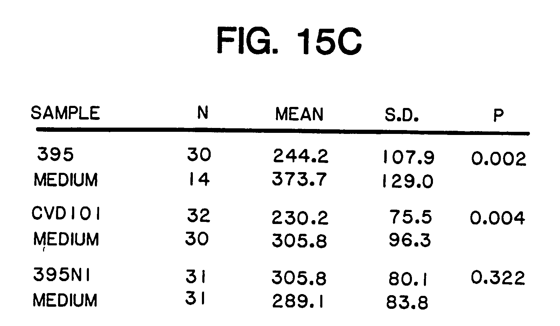

Variation in transepithelial conductance reflects modification of tissue permeability through the intercellular space, since plasma membrane resistances are relatively high. Since ZO represents the major barrier in this paracellular pathway and variation in Gt is the most sensitive measure of ZO function, morphological modifications of ZO induced by V. cholerae 395, CVD101 and 395N1 supernatants were examined. If a low-molecular-weight electron-dense marker such as wheat germ agglutinin - horseradish peroxidase (WGA-HRP) is added to the mucosal side of an epithelial sheet, it will usually not pass beyond to ZO [Alberts, B. et al., Molecular Biology of the Cell 2nd ed (1989)]. WGA-HRP was added to the mucosal side of intestinal tissue treated with culture supernatants of 395, CVD101 , 395N1 or uninoculated broth control for 60 minutes. As seen in Figure 14, tissues treated with uninoculated culture medium were not permeable to WGA-HRP (Fig. 14A), while 395 and CVD101 -treated tissues showed the entry of the stain into the paracellular space (Fig. 14b and 14d). Tissues exposed to 395N1 supernatants were unaffected, inasmuch as the intercellular space remained tight enough to exclude the passage of WGA-HRP (15C). These results were confirmed and extended using freeze-fracture electron microscopy wherein the number of strands lying in parallel at the ZO correlates with transepithelial electrical conductance. Tissues exposed to culture supernatants showed a mixture of unaltered ZO (Figure 15A) and altered ZO with decreased strand complexity (Figure 15B). Strands lying perpendicular to the long axis of the ZO appeared to be preferentially lost, resulting in a decreased number of strand intersections. The complexity of the ZO exposed to each strain supernatant was quantified by measuring the density of strand intersections. As seen in Figure 15C, tissues treated with culture supernatants of 395 or CVD101 showed a significant decrease in the number of strands and in the complexity of the reticulum of the ZO when compared to tissues treated with uninoculated broth or supernatants of 395N1.

The alterations of ZO morphology induced by 395 and CVD101 parallel the increased tissue conductance induced by these strains. The function of intestinal ZO is to regulate the paracellular pathway and restrict or prevent the diffusion of water-soluble molecules through the intercellular space back into the lumen. This diffusion is driven by concentration gradients created by the transepithelial transport processes. As a consequence of alteration of the paracellular pathway, intestinal mucosa becomes more permeable and water, Na and Cl leak into the lumen, resulting in diarrhea. The alteration of the paracellular pathway induced by V. cholerae 395 and CVD101 is specific for the small intestine; substitution of rabbit cecal tissue for ileal tissue resulted in no variation in Gt induced by 395 supernatant (data not shown). This is the first report of a bacterial factor which is capable of loosening tight junctions in intact intestinal tissue and may represent a new mechanism of bacterial diarrhea. Clostridium difficile toxin A, influenza, and vesicular stomatitis (VSV) viruses have been shown to loosen tight junctions in tissue culture monolayers but such activity in intact tissue or correlation with diarrhea have not been reported.

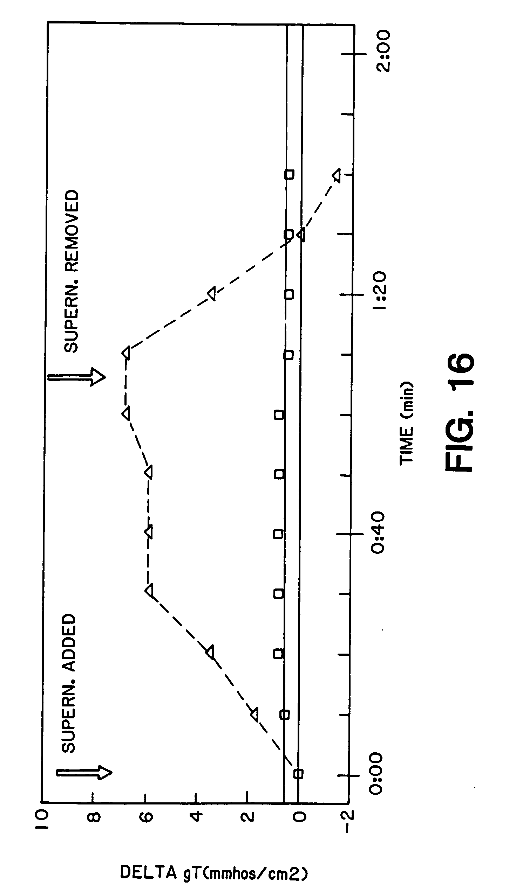

Thus, V. cholerae 395 and CVD101 produce a factor which may be responsible for diarrhea seen in volunteers ingesting ctx deletion mutants of V. cholerae. The diarrhea induced by these ctx mutants is equivalent to that seen with many strains of enterotoxigenic E. coli. This secretogenic factor, which applicants have termed ZOT for zonula occludens toxin, induces an early increase in Isc and tissue conductance which is not related to the effects of CT on ion fluxes. This increase in Gt is associated with loosening of the tight junctions, an effect which was quickly reversed upon removal of the supernatant (Fig. 16). The quick reversal of this effect is in contrast to the long-lasting effect of CT. These results do not account for previously unexplained observations of Fields, et al., J. Clin. Invest. 51, 796-804 (1972) who noted an immediate increase in Isc induced by crude, but not purified CT preparations, and may account for Nishibuchi et al., Infect. Immun. 40, 1083-1091 (1983) who noted an early fluid accumulation (FA) unrelated to the delayed CT-induced FA in suckling mice fed V. cholerae. The ability of CT-negative V. cholerae to induce diarrhea in volunteers correlates with production of ZOT by two attenuated strains derived from the same parent strain; CVD101 (diarrheagenic) produces ZOT while 395N1 (non-diarrheagenic) produces little or no ZOT activity.

Another culture of Vibrio cholerae comprises a Vibrio cholerae strain having a region of chromosomal DNA coding for cholera toxin and zonula occludens toxin deleted, and having inserted a mercury resistance gene and DNA coding for B subunit of Vibrio cholerae toxin. A method of isolating such deletion mutants is also described comprising the steps of:
(a) constructing a plasmid comprising Vibrio cholerae sequences coding for cholera toxin and zonula occludens toxin and a gene for a selectable marker of foreign origin, wherein said plasmid is incapable of replicating extrachromosomally in Vibrio cholerae;
(b) mating a microorganism carrying said plasmid with a virulent strain of Vibrio cholerae containing said sequences coding for cholera toxin and zonula occludens toxin inserted between flanking identical copies of a second sequence of sufficient length to promote detectable in vivo recombination;
(c) selecting for Vibrio cholerae expressing said selectable marker;
(d) growing the selected product of (c) in the absence of the selective agent;
(e) selecting for Vibrio cholerae which no longer express the selective marker, and therefore have a region of the chromosomal DNA coding for cholera toxin and zonula occludens toxin deleted;
(f) constructing a second plasmid comprising a mercury resistance gene and DNA coding for B subunit of Vibrio cholerae toxin and a gene for a second selectable marker of foreign origin wherein said plasmid is incapable of replicating extrachromosomally in Vibrio cholerae, and wherein sequences of sufficient length to promote detectable in vivo recombination flank said mercury resistance gene and DNA coding for B subunit of Vibrio cholerae toxin;
(g) mating a microorganism carrying said second plasmid with said Vibrio cholerae recited in step (e) containing sequences homologous to said sequences of sufficient length to promote detectable in vivo recombination;
(h) selecting for Vibrio cholerae expressing said second selectable marker;
(i) growing the selected product of step (h) in the absence of the second selective agent;
(j) selecting for Vibrio cholerae which no longer express the second selective marker; and
(k) screening said Vibrio cholerae recited in step (j) for Vibrio cholerae that have a mercury resistance gene and DNA coding for B subunit of Vibrio cholerae toxin and have a region of chromosomal DNA coding for cholera toxin and zonula occludens toxin deleted.

This method for isolating deletion mutants of Vibrio cholerae having a region of chromosomal DNA coding for cholera toxin and zonula occludens toxin deleted, and having inserted a mercury resistance gene and DNA coding for B subunit of Vibrio cholerae may use in step (f) flanking sequences of sufficient length comprising a gene that can be disrupted without affecting colonization and immunity of Vibrio cholerae. An example is the hemolysin gene. V. cholerae CVD110 was constructed according to this method, and has a region of chromosomal DNA coding for A and B subunits of cholera toxin and zonula occludens toxin deleted, and has a mercury resistance gene and DNA coding for B subunit of Vibrio cholerae toxin inserted at the site of hemolysin gene. Other examples of sequences of sufficient length comprise the his gene (Hone, Microbial Pathogenesis 5, pp. 407-478 (1989)) and the nanH gene (Vimr, J. of Bacteriology 170, pp. 1495-1504 (1988)).

In the examples that follow, any of the techniques, reactions, and separation procedures are already well known in the art. All enzymes, unless otherwise stated, are available from one or more commercial sources, such as New England BioLabs--Beverly, Massachusetts; Collaborative Research--Waltham, Massachusetts; Miles Laboratories--Elkhart, Indiana; Boehringer Biochemicals Inc.--Indianapolis, Indiana; and Bethesda Research Laboratory--Rockville, Maryland, to mention a representative few. Buffers and reaction conditions for restriction enzyme digestion are used according to recommendations supplied by the manufacturer for each enzyme, unless indicated otherwise. Partial digestions with restriction enzymes are carried out using a reduced enzyme concentration which must be predetermined from preliminary experiments for each enzyme batch. Standard methodology for other enzyme reactions, gel electrophoresis separations, and E. coli transformation may be found in Methods in Enzymology Volume 68, Ray Wu, editor, Academic Press (1979). Another standard reference is Maniatis, T. et al. Molecular Cloning, Cold Spring Harbor (1982). Bacteria were grown according to procedures generally described in Miller, Experiments in Molecular Genetics, Cold Spring Harbor Laboratory (1972) Vibrio cholerae were propagated according to procedures generally described in Lennett, E.A. et al., eds., Manual of Clinical Microbiology 3rd Edition, American Society of Microbioloy, Washington (1980). E. coli and V. cholerae were mated according to procedures generally described in Johnson, Steven R. et al. J. Bact. 137, 531 (1979); and Yokata, T. et al. J. Bact. 109, 440 (1972).

The strains of this invention have been deposited at the American Type Culture Collection, located in Rockville, Maryland, prior to execution of the present application. The strains deposited are V. cholerae JBK56, V. cholerae JBK70, V. cholerae N16961, V. cholerae JPK70 (pLIBK5l), V. cholerae Ogawa 395, CVD101, CVD109, V. cholerae E7946, and E. coli SM10 lamda pir pCVD51, V. cholerae CVD110, and E. coli SY327 lamda pir (pCVD622.2B), which have ATCC accession numbers 39,317, 39,318, 39,315, 39,316, 39,541, 39,540, 55,057, (deposited June 4th, 1990), 55,056 (deposited June 4th, 1990), 68,335 (deposited June 5th, 1990), 55188 (deposited June 3rd, 1991), and 68630 (deposited June 3rd, 1991, respectively).

### Example 1

### Construction of a Plasmid Having a Selectable Marker Gene Inserted to Replace the Toxin Genes

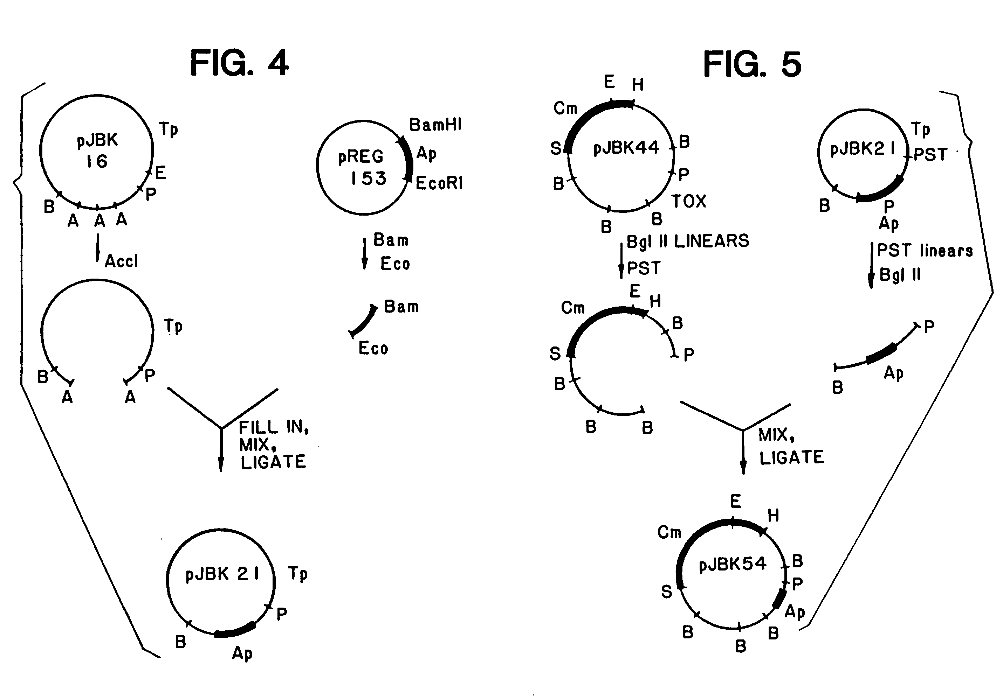

The plasmid JBK16 contains a 4 kb PstI-Bg1 II fragment of the chromosome containing the toxin genes. The toxin genes are flanked by Acc I sites and contain an internal Acc I site. JBK16 was digested to completion with Acc I and the Acc I fragments containing the toxin genes were separated from the rest of the plasmid. The remaining overlapping or "sticky" Acc I ends were made blunt-ended by "filling in" with the Klenow fragment of E. coli polymerase (i.e., the single-stranded DNA remaining after Acc I digestion were made double-stranded with flush ends). A gene encoding ampicillin resistance was purified from the plasmid pREG153 (pREG153 is a derivative of pREG151 [Weiss, A. et al. J. Bact. 152, 549-552] altered by substitution of ampicillin resistance for trimethoprin resistance and addition of cos sequences) and the "sticky" ends "filled in" as above. This fragment was then ligated to the vibrio DNA so that the Ap resistance genes were in exactly the same place as the now-deleted toxin genes, flanked by the same vibrio sequences. The resulting plasmid was designed pJBK21 (Figure 4) containing the deletion toxin region and the Ap resistance gene.

### Example 2

### Addition of Flanking Homologenous Sequences, Followed by Conjugal Gene Transfer into V. Cholerae

To insure the specific insertion into the chromosome of the deletion in pJBK21, approximately 7,000 bp of additional DNA was added to each end of the Pst-Bgl II fragment from pJBK21. (The probability of the homologous recombination event occurring increases with increasing length of flanking homologous sequences.) To achieve this, an approximately 18 kb fragment was cloned from the chromosome of N16961. This clone was designated pJBK44 and contains the 4 kb Pst-Bgl tox gene fragment flanked by approximately 7kb of DNA on each side (see Figure 5). The plasmid pJBK21 was partially digested with Pst I so that only one of the Pst sites would be cut (an additional Pst side was added within the ampicillin resistance gene) followed by digestion with Bgl II to isolate the 4 kb Pst-Bgl II fragment containing the deletion toxin region and the Ap resistance region. The plasmid pJBK44 containing the ca 18 kb vibrio fragment was partially digested with Bgl II so that only one of the 4 Bgl II sits present would be cut. This partial digestion wa followed by complete digestion with Pst I and the resulting fragments separated by electrophoresis through 0.3% agarose. The separated fragments were then purified and analyzed and one fragment was found which contained all of the sequences of pJBK44 except for the 4 kb. Pst-Bgl tox gene fragment (see Figure 5.). This fragment representing the flanking DNA was then mixed ligated to the Pst-Bgl fragment from pJBK21 containing the ampicillin resistance. The resulting plasmid, pJBK54, contained approximately 17 kb of Vibrio chromosome with an ampicillin resistance gene substituted for the deleted toxin genes.

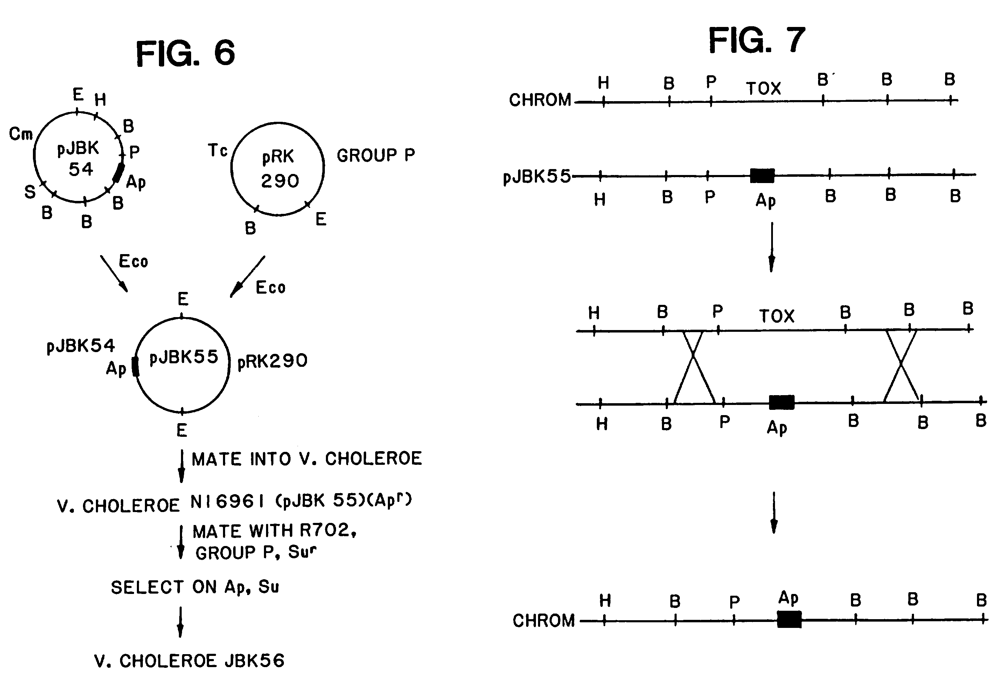

The modified chromosomal region was the cloned into a plasmid which can be readily mobilized in V. cholerae. The plasmid pRK290 [Ditta, G. et al. Proc. Nat. Acad. Sci. 77, 7347 (1980)] belongs to the plasmid incompatibility group P and possesses a single Eco RI site into which pJBK54 was cloned (Figure 6). The resulting plasmid pJBK55 was then mated into V. cholerae N16961 using the conjugative plasmid pRK2013, yielding V. cholerae N16961 (pJBK55) (Ap^{r}).

### Example 3

### Recombination in vivo

The mutant toxin genes, after conjugal gene transfer as described in Example 2, now existed extrachromosomally in V. cholerae strain N16961 (see Figure 1). At a very low frequency (perhaps 10⁻⁶ to 10⁻⁸) the homologous flanking sequences base pair and cross over into the chromosome (see Figure 7). This rare event will result in the substitution of the deleted toxin region on the plasmid for the ctx genes on the chromosome. To select for this rare event, the plasmid incompatibility phenomenon was exploited [Ruvkin, G.B., supra]. Plasmids can be divided into incompatibility groups, designated A through W, on the basis of their ability to be stably maintained together in the same cell. If two plasmids cannot be stably maintained together in the same cell, they are incompatible and belong to the same incompatibility group presumably because they utilize the same replication mechanism in the cell. By selectively using an antibiotic resistance present on one plasmid but not on the other, it is possible to select which of two incompatible plasmids will be maintained The plasmid pJBK55, because of its pRK290 origin, belongs to the (Inc) group P. The plasmid R702 also belongs to the Inc P group and encodes resistance to kanamycin, tetracycline, sulfonamide, and streptomycin, but not ampicillin. By mating pR702 (Su^{R}) into N16961(pJBK55) (Ap^{R}) and selecting on media containing both ampicillin and sulfonamide, selection was made for cells in which the ampicillin resistance had been incorporated into the chromosome and sulfonamide resistance remains on the plasmid R702, since pR702 and pJBK55 are incompatible (see Figure 2). The resultant strain JBK56 (Figure 3) was ampicillin resistant, and toxin negative when tested in Y-1 adrenal cells and by Gm₁ ELISA. Furthermore, when chromosomal DNA was hybridized to DNA probes containing clone cholera toxin (CT) genes, JBK56 was negative, suggesting that the toxin genes were completely deleted.

The antibiotic resistance encoded on R702 was eliminated by selecting a spontaneously cured derivative lacking the plasmid (this occurred at a frequency of about 1 in 2,000).

### Example 4

### Elimination of the Selectable Marker of Example 1

To eliminate the ampicillin resistance, a derivative of pJBK55 was constructed in which genes encoding resistance to mercury (Hg) from R100 were cloned into the Pst site of the Ap gene, thereby insertionally inactivating the ampicillin resistance. This derivative was then mated into V. cholerae JBK56, followed by pR702 and selection made as above for Hg^{R}, Ap^{S} V. cholerae. The final strain, V. cholerae JBK70, is sensitive to all antibiotics tested, resistant to mercury, and phenotypically toxin negative. Its chromosomal DNA did not detectably hydridize to DNA probes containing CT genes. Short of sequencing the DNA for the entire chromosome, JBK70 appears to be unaltered from the parent strain N16961 except for the deletion of the toxin genes and insertion of mercury resistance and inactive ampicillin resistance genes. Such a strain cannot revert to toxigenicity because the toxin genes are not merely mutated but are completely deleted.

### Example 5

### Conjugal Gene Transfer to Confer Antitoxic Immunity

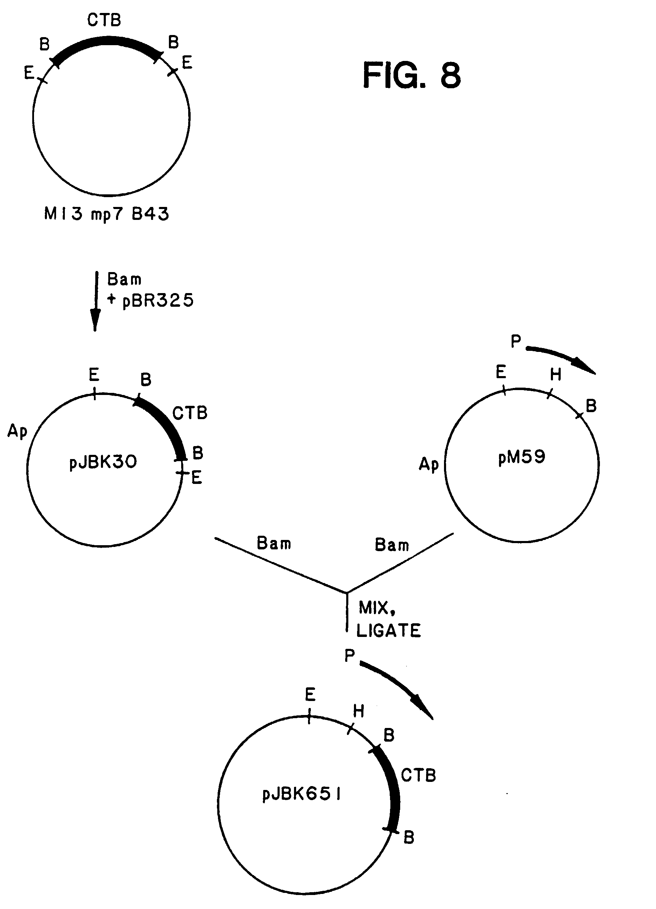

If both antibacterial immunity and antitoxic immunity are desired for synergy, a derivative of JBK70 can be made to produce the B subunit of cholera toxin only. To accomplish this end, a toxin derivative was made that produces B only and lacks the genes for A (Figure 8). A Hpa II fragment from pJBK16 containing the B structural gene was cloned into a phage cloning vector, M13mp7 placing a Bam HI and an Eco RI site on either side of the gene (Figure 8). The fragment, now flanked by Bam HI sites was cloned into pMS 9 which contains the very strong trp promoter. The placing of the B genes under the transcriptional control of a strong promoter insures high production of B antigen. Of the clones examined, approximately 50% produced no antigen. This finding reflects the two possible orientations for the cloned insert--one forward, one backward. One derivative, pJBK51, which produced B subunit was mated into Vibrio cholerae JBK70 and found to produce even more B antigen then the parent strain N16961, yielding JBK70 (pJBK51). Other B-only mutants have been created using different promoters, including the P_{L} promoter and these can be evaluated in appropriate models for any significant in vivo expression differences.

### Example 6

### Colonization of Infant Mouse Intestine with JBK70 without Reversion to Toxigenicity

Suckling mice (2.0-3.5g.) were removed from their mothers and starved for 3 to 8 hours. Four of them were then inoculated on day 1 per os to stomach using a 22g animal feeding needle. The inoculum was about 10⁸ CFU (colony-forming units)/mouse of JBK70 in a volume of between 0.05 ml and 0.1 ml. The inoculum was prepared in BHI broth essentially as described in Baselski, V. et al, supra. The inoculum contained about 0.01% Evans blue dye. The presence of this dye in the stomach, seen through the abdominal wall, indicated proper delivery of the inoculum. Addition of Evans blue dye was discontinued after day 1 (see Table I), to avoid inhibition of JBK70.

Subsequent inoculations involved mouse-to-mouse (MXM), or alternatively, mouse-to-plate-to-mouse (MXPXM), but required different procedures to prepare the inoculum compared to the Baselski protocol for the inoculation on day 1.

To prepare MXM inoculum, the gut was dissected from stomach to anus under sterile precautions. The gut was weighed, placed in a glass homogenizer tube, and about 0.5 ml BHI broth added. The mixture was homogenized briefly with a Teflon pestle until tissue was liquified. The resulting suspension was used to inoculate about 10⁻⁸ CFU into each infant mouse. It was checked for purity by streaking on MEA (meat extract agar) plates. No Evans blue dye was added.

To prepare MXPXM inoculum, a sterile loop was used to transfer cells from an MEA plate to BHI broth. About 10¹1 CFU/ml were added to about 1 ml of BHI so that a dense suspension was formed. The mixture was vortexed to homogeneity, and 0.05-0.1 m. (about 10¹0 CFU) inoculated per os into each infant mouse. No evans blue dye was added.

For all inoculations, mice were held in beakers at room temperature of 73-76°F. Beakers were placed in a plastic box which was loosely covered in order to maintain the mice at slightly about ambient temperature, about 78°F.

As the results in Table I indicated, there were sufficient cells in the intestine to inoculate the next animal, as checked by streaking on MEA plates. The Vibrio cholerae JBK70 therefore colonized the gut of infant mice. Furthermore, the fluid accumulation levels did not increase since there were no substantial increases in the FA ration

**TABLE I:**

| MOUSE PASSAGE HISTORY OF JBK70 | | | | | | | | |
|---|---|---|---|---|---|---|---|---|
| (24 HOUR SCHEDULE) | | | | | | | | |
| DAY | MXM | D/T | FA RATIO | SHAM D/T | MXPXM | D/T | FA RATIO | COMMENTS |
| 1 | | | | | | | | at 6p.m. inoc. mice with 10⁸-10⁴ CFU JBK70 from 24 h plate (Evans Blue Dye was used) |
| 2 | MX1 | 2/4 | 0.038 | | | | | |
| noon | | | (avg of 2) | | | | | |
| 3 | MX2 | 1/4 | 0.051 | | | | | |
| noon | | | 0.049 | | | | | |
| | | | 0.043 | | | | | |
| 4 | MX3 | 2/3 | 0.050 | | MXPXM | 2/3 | 0.040 | |
| noon | | | | | 1 | | | |
| 5 | MX4 | 0/5 | 0.043 | 0/5 | | | | |
| 1 P.M. | | | 0.044 | | | | | |
| | | | 0.052 | | | | | |
| 6 | MX5 | 1/4 | 0.053 | | | | | |
| 1 P.M. | | | 0.064 | | MXPX | 0/3 | 0.060 | |
| | | | 0.053 | | 2 | | 0.054 | |
| 7 | MX6 | 1/3 | 0.063 | | | | | |
| 3 P.M. | | | 0.042 | | | | | |
| 8 | MX7 | 1/3 | 0.049 | | MXPXM | 0/3 | 0.045 | |
| 3 P.M. | | | 0.050 | | 3 | | 0.051 | |
| | | | | | | | 0.059 | |
| 9 | MXM | 2/3 | 0.054 | | | | | |
| | 8 | | | | | | | |
| 10 | MXM | 0/3 | 0.041 | | MXPXM | 1/3 | 0.048 | |
| | 9 | | 0.050 | | 4 | | 0.044 | |
| | | | 0.032 | | | | | |
| 11 | MXM | 0/3 | 0.050 | | | | | |
| | 10 | | 0.054 | | | | | |
| | | | 0.055 | | | | | |
| 12 | MXM | 2/3 | 0.037 | 0.2 | MXPXM | 1/3 | 0.049 | |
| | 11 | | | | 5 | | 0.048 | |
| XM = Passage from Mouse to Mouse D/T = Deat Total FA Ratio = weight of gut/(weight of carcass - gut) MXPXM = Passage from Mouse to Plate to Mouse SHAM animals were given sterile BHI broth. | | | | | | | | |

(an FA ratio greater than or equal to 0.065 is a positive fluid accumulation). Evidence of reversion to toxigenicity would have indicated otherwise.

### Example 7

### Construction of V. cholerae strain CVD101 having a Restriction Fragment Deletion within the Gene coding for the A Subunit

Another classical strain chosen for attenuation was Vibrio cholerae Ogawa 395 (alternatively designated "395") which, like N16961, has been extensively studied in volunteers and confers solid immunity [Levine, M.M. "Immunity to cholera as evaluated in volunteers," in Cholera and Related Diarrheas: 43rd Nobel Symposium, Stockholm 1978. (O. Ouchterlong & J. Holmgren, eds.) Basel: S. Karger, pp. 195-2-3 (1980); Levine, M.M et al. Acute Enteric, supra (1981)]. The procedure employed in the attenuation of 395 was not substantially different from that employed for N16961 (as described in Examples 1-5).

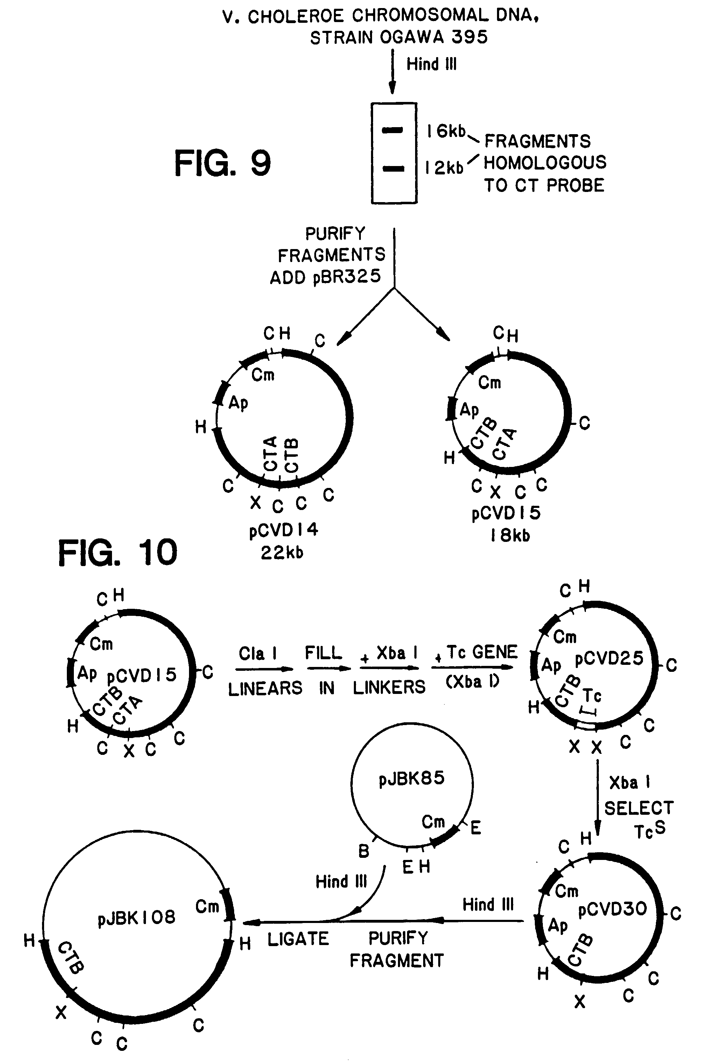

The first step involved the cloning and mapping of the two toxin gene copies of 395. Southern blot analysis revealed two Hind III fragments of about 16 and about 12 kb in length, both of which hydridized with cloned cholera toxin genes. These fragments were purified by agarose gel electrophoresis and cloned into alkaline phosphates treatedHind III digested pBR325 (Figure 9). The resulting recombinant plasmids containing the toxin genes were designated pCVD14 and pCVD15.

Plasmids pCVD14 and pCVD15 were then mapped with restriction endonucleases. An Xba I-Cla I fragment of about 550bp was found, containing the entire base sequence of the A₁ subunit with the exception of codons for the first 10 amino acid residues of A₁. This Xba I-Cla I fragment was deleted in vitro from both pCVD14 and pCVDl5 in a series of steps as shown in Figure 10 for pCVD15. First, partial digestion with Cla I yielded a population of linear molecules in which only one of five Cla I sites was cut. Next, the ends of the linear molecules were made blunt-ended by filling in with DNA polymerase. Xba I linkers were ligated onto the blunt-ended Cla I sites yielding a collection of molecules in which an Xba I enzyme was then added to trim the linker and a tetracycline resistance gene on an Xba I fragment was added and ligated. After transformation into E. coli K-12 and selection on tetracycline, the plasmid content of a number of transformants was examined. A variety of deletion mutations were found in which one or more Xba I-Cla I fragments were deleted. One deletion mutant was chosen which lacked only the 550 bp Xba I-Cla I fragment containing the A₁ gene. This deletion mutant, designated pCVD25 was purified, digested with Xba I and relegated to delete the tetracycline resistance gene. The resulting clone, PCVD30, was negative for holotoxin as measured in Y-1 adrenal assay [Sack, D.A. et. al. supra (1975)], but positive for production of B subunit, as measured by ELISA [Sack, D.A. et al. supra (1980)], and lacked the genes for A₁, as shown by DNA hydridization using labeled A₁ probe. The Hind III fragment of pCVD30 containing the toxin deletion mutation was then cloned into pJBK85, a Tc sensitive, Cm resistant derivative of pJBK108. The resulting plasmid was designated pJBK108.

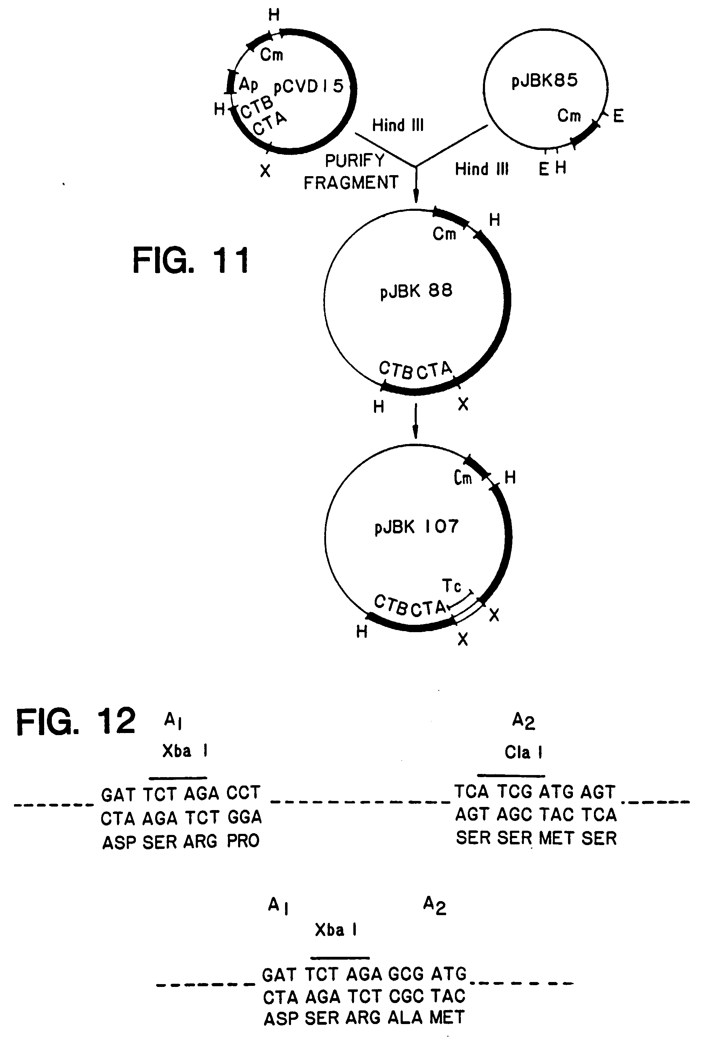

The lack of a selectable marker in the toxin deletion mutation in pJBK108 necessitated a modification of the method previously used to attenuate El Tor N16961. To accomplish the deletion of the A₁ genes from 395, the Hind iii fragment from pCVD15 was cloned into pJBK85, resulting in pJBK88 (Figure 11). The tetracycline resistance gene on an Xba I fragment was then cloned into the Xba site within the A₁ gene of PJBK88, yielding pJBK107. This tetracycline resistance was then recombined into the chromosome of 395 as previously done for V. cholerae pJBK56. pJBK107 (Tc^{r}, Cm^{r}) was mobilized into 395 and a second Inc P plasmid, pR751 (Tp^{r}) was introduced. Selection of Tc^{r}, Tp^{r}, Cm^{s} colonies resulted in V. cholerae JBK113, which contained tetracycline resistance genes in both chromosomal toxin gene copies. pJBK108, containing the deletion mutation, was then mobilized into V. cholerae JBK113. Homologous recombination of the deletion mutation into the chromosome will result in the loss of the A₁ gene sequences, an event which can be detected by loss of tetracycline resistance. Because the recombination even occurs at a very low frequency, an enrichment procedure for tetracycline sensitive cells in a population of tetracycline resistant cells was employed. This enrichment procedure exploited the fact that tetracycline is a bacteriostatic antibiotic whereas ampicillin and D-cyclo-serine are bactericidal. Therefore, a culture of V. cholerae JBK113 containing pJBK108 was grown for 3 hr at 37° in L-broth containing 2 micro g/ml tetracycline, 50 micro g/ml ampicillin and 50 micro g/ml D-cycloserine. At the end of 3 hours, most of the tetracycline resistant cells were killed, and tetracycline sensitive cells were detected by plating onto L-agar and replica plating onto L-agar with tetracycline. Tetracycline sensitive colonies were probed for the presence of A₁ genes by DNA hybridization. One tetracycline sensitive strain having deletions for both gene copies of the A₁ subunit was designated V. cholerae CVD101 and tested for production of B subunit by ELISA [Sack, supra]. V. cholerae CVD101 was found to produce B subunit antigen at levels substantially equivalent to the toxigenic parent V. cholerae 395.

### Example 8

### DNA Sequencing of the Toxin Genes

The entire DNA sequence of the toxin genes of V. cholerae Inaba 62746 has been determined, part of which has been reported in Lockman et al., J. Biol. Chem. 258, 13722 (1983). The restriction endonucleases mapping of pCVD14 and pCVD15 indicates that the sequences found in strain 62746 are also present in the toxin genes of 395. The predicted junction after deletion of the 550 bp Xba I-Cla I fragment, but with addition of an Xba I linker sequence, is shown in Figure 12. The Xba I site of the cholera toxin sequence spans amino acid residues 10 and 11 of the A₁ structural gene (not counting the 18 amino acid leader sequence for A₁). The Cla I site of the sequence is located at the last residue of A₁ and the first residue of A₂.

### Example 9

### Construction of V. Cholera Strain Having A Zonula Occludens Toxin Deletion in CVD101

A zot deletion mutant of V. cholerae is prepared in the same way as the CVD101 cholera toxin deletion mutant described in Example 7. The zot gene is contained in the recombinant plasmid pBB68. pBB68 consists of an EcoRI-Pbr I chromosomal DNA fragment from V. cholerae S69B which contains the zot gene and ctx genes which have a deletion of a 550bp XbaI-claI fragment. A Stu I -Acc I restriction fragment of 575 base pairs is deleted in vitro from pBB68 by digesting with the restriction enzymes Stu I and Acc I, and making the ends of the molecules blunt-ended by filling in with DNA polymerase. (This will remove 48% of the 1199 base pair zot gene.) One half of this sample is ligated onto itself, making a deletion mutant. The other half of this sample is ligated to a tetracycline resistance gene (of foreign origin), thus giving a selective marker.

The zot deletion mutant constructed in vitro above is introduced into the chromosome of V. cholerae CVD101 as previously described for the construction of the ctx deletion mutant of CVD101. The tetracycline resistant clone derived above is cloned into the Inc P Plasmid pJBK85. This plasmid (Tc^{r} Cm^{r}) is mobilized into CVD101, selecting for Tc^{r}. A second Inc P plasmid, pR751 (Tp^{r}) is introduced. Selection of Tc^{r}, Tp^{r}, Cm^{s} colonies result in V. cholerae strains in which the Tc^{r} gene has recombined into the zot gene.

The plasmid containing the Stu I - Acc I deletion mutant without the Tc^{r} gene is then mobilized into the Tc^{r} V. cholerae strain. Homologous recombination of the deletion mutant into the chromosome results in the loss of the zot gene sequences, an event which can be detected by loss of Tc^{r}. Tc^{s} colonies are selected and screened for loss of the zot sequences by DNA hybridization using the Stu I - Acc I fragment as a probe.

### Example 10

### Construction of CVD109- a V. Cholera Strain Having Restriction Fragment Deletions of Sequences coding for V. Cholera Toxins and for Zonula Occludens Toxin

The construction of attenuated V. cholerae strain CVD109 involves the introduction of cloned Vibrio sequences along with sequences encoding a selectable marker into the chromosome of a virulent V. cholerae strain. An initial in vivo recombination event of homologous sequences from the recombinant plasmid into the chromosome provides a selectable marker at this site. A second in vivo recombination event between homologous flanking sequences results in excision of proficient genes from the chromosome with the end product being a deletion mutation.

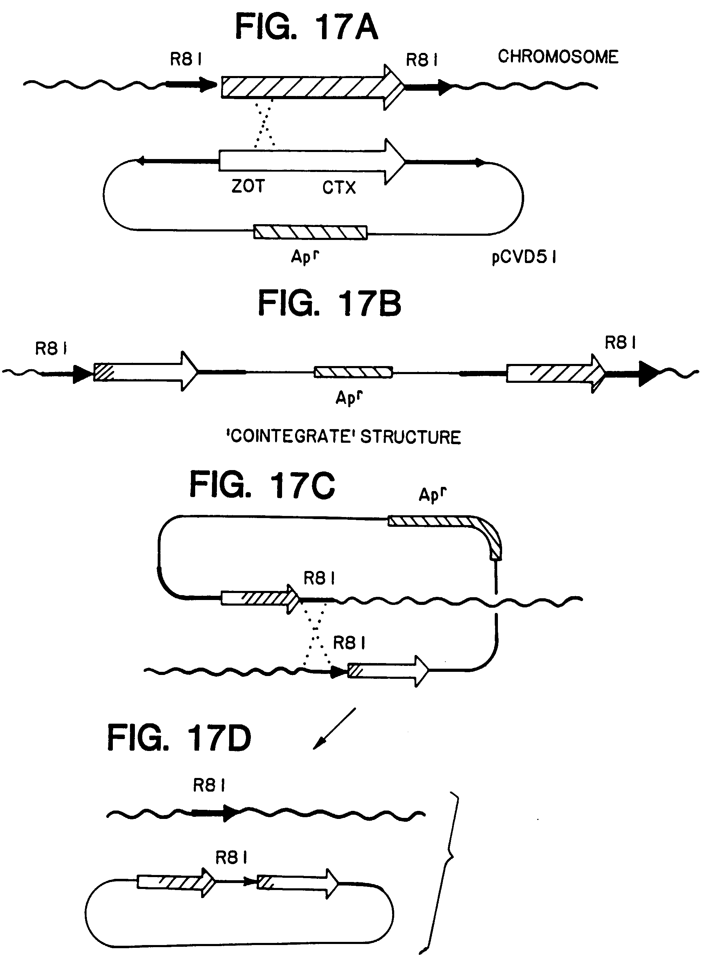

Figure 17 illustrates the construction of CVD109. The zot and ctx genes are adjacent to each other on the V. cholerae chromosome. Multiple copies of a 2700 base pair DNA sequence called RS1 (for repetitive sequence) are on both sides of the zot and ctx genes in virulent V. cholerae strain E7946 (El Tor biotype, Ogawa serotype). In Fig. 17A, the zot and ctx sequences are shown by a large open or hash-marked arrow. RS1 sequences are shown by a smaller, solid arrow.

The recombinant plasmid, pCVD51 (Figure 17A), contains cloned zot/ctx sequences (open arrow) which are homologous to the chromosomal zot/ctx sequences (shown by hash-marked arrow in Figure 17A) and contains a selectable marker, ampicillin resistance (Ap^{r}). The plasmid vector into which the Vibrio sequences were cloned is pGP704 (Miller and Mekalanos J. Bacteril, 170, 2575-2583 (1988)). This plasmid cannot replicate extrachromosomally in V. cholerae but can replicate in permissive E. coli strains. pCVD51 was mated from E. coli to V. cholerae E7946. Since this plasmid cannot replicate extrachromosomally in V. cholerae, selection of Ap^{r} colonies yielded strains in which the entire plasmid, with the Ap^{r} marker, was homologously recombined into the chromosome at the site of the zot/ctx sequences. (The exact site of recombination, whether in the zot or ctx gene, is not known.) The result of this single cross over (not double cross over) event is termed a "cointegrate" structure and is depicted in Figure 17B.

The RS1 sequences flanking the zot/ctx region are of sufficient length to provide detectable in vivo recombination; intra-molecular recombination between the homologous RS1 elements results in the loss of all sequences between them. The Ap^{r} V. cholerae with the integrated plasmid was grown in the absence of ampicillin and the Ap sensitive (Ap^{s}) colonies were selected. Recombination of the RS1 elements flanking ctx and zot resulted in the loss of the intervening zot and ctx sequences along with the plasmid vector containing Ap^{r} (Figure 17C).

The Ap^{s} V. cholerae colonies resulting from the above steps were screened by DNA hybridization for zot sequences. The DNA probe consisted of a 575 base pair Stu I - Acc I restriction fragment derived from the cloned zot gene. Colonies which did not hybridize to this probe were selected and probed for the presence of ctx genes by DNA hybridization using a ctx gene probe. These hybridization results confirmed the loss of both the zot and ctx genes. One representative strain was saved and designed CVD109. Figure 17D depicts the chromosome of CVD109 which is deleted of zot and ctx sequences but retains one copy of the RS1 element. (The plasmid shown in 17D is not retained in the final Ap^{s} strain but is depicted only to illustrate the outcome of the second cross-over event. This transient product is spontaneously lost since the plasmid cannot replicate extrachromosomally in V. cholerae.)

### Example 11

### Construction of CVD110-a V. Cholerae Strain Having Restriction Fragment Deletions of Sequences Coding For A and B Subunits of V. Cholerae Toxin and for Zonula Occludens Toxin, and Having Inserted a Mercury Resistance Gene and DNA Coding for B Subunit of V. Cholerae Toxin

CVD110 was constructed directly from V. cholerae CVD109, the description of which has already been provided. V. cholerae CVD109 lacks both the A and B subunits of cholera toxin (CT) as well as the gene encoding Zot and is sensitive to mercury. A gene fragment that contains the CT B subunit gene (ctxB) and a mercury resistance gene was constructed in vitro. This construction was then inserted into the chromosome of CVD109, specifically into the hemolysin gene. Thus, the final vaccine strain, CVD110, produces the B but not the A subunit of cholera toxin, is resistant to mercury and does not produce wild type HlyA protein (hemolysin).

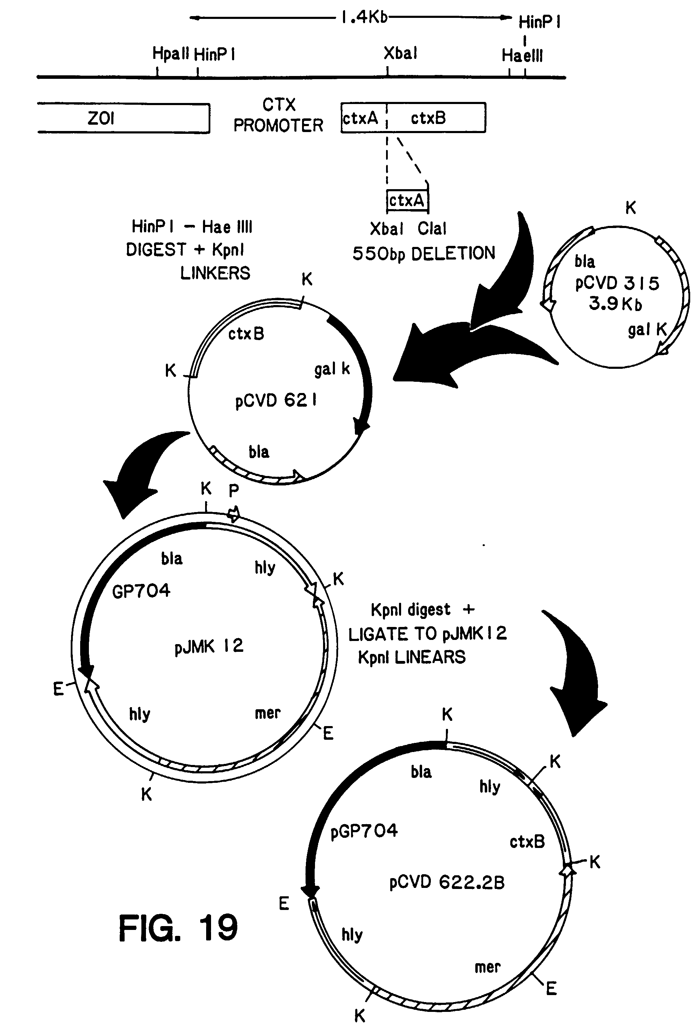

CT B construction: The ctxB and ctx promoter sequences were obtained from plasmid pCVD30, which is described in Example 7. This plasmid pCVD30 contains a deletion of the ctxA gene. A 1.4 kilobase fragment containing the ctxB gene and the ctx promoter but not the zot gene was obtained by digesting pCVD30 with Hin P1 and Hae III enzymes. The fragment was treated with T4 DNA polymerase to render the ends of this fragment blunt-ended and synthetic KpnI linkers were ligated to this fragment. The fragment was then cloned into the vector pCVD315 [Galen, et al. Advances in Research on Cholera and Related Diarrheas, vol. 7 (Sack et al., Eds.) pp. 143-153 (1990)] Vector pCVD315 has no particular significance for this purpose other than the presence of a Kpn I site. The resulting plasmid containing the ctxB gene was called pCVD621 (Figure 19).

Mercury resistance genes: The source of the mercury resistance genes (mer) was the same as that used for V. cholerae JBK70. A 4.2 kb Nco I - Stu I fragment containing mer was originally derived from pDB7 [Barrineau, et al. J. Molecular & Applied Genetics (1984) vol. 2, pp.601-619]. The fragment was treated with DNA polymerase (Klenow fragment) to render the ends of this fragment blunt-ended and synthetic Kpn I linkers were ligated to this fragment. The fragment was then cloned into plasmid pCVD43.2 (unpublished), which is a derivative of pCVD43 [Kaper, et al. Advances in Research on Cholera and Related Diarrheas, vol. 6 (Ohtomo, et al., Eds.) pp. 161-167 (1988)]. pCVD43 contains the cloned hemolysin genes (hlyA) of V. cholerae without a 400 bp Hpa I fragment internal to hlya. The deletion of the 400 bp Hpa I fragment renders the gene inactive [Kaper, et al. Advances, etc. vol. 6]. pCVD43.2 is identical to pCVD43 except that a synthetic Kpn I linker has been ligated into the single Hpa I site of pCVD43. The combined clone of the mer genes inserted into the hylA gene is called pCVD43.3.

Insertion of ctxB and mer genes into CVD109: To introduce these genes into the chromosome of CVD109, plasmid vector pGP704, which is described in Example 10, was used. An 8.1 kb Cla I - Bgl II fragment from pCVD43.3 containing mer and hylA was cloned in pGP704 to produce pJMK12 (Figure 19). pJMK12 was partially digested with Kpn I to yield a population of linear molecules in which only one of 3 Kpn I sites was cut. The 1.4 kb fragment of pCVD621 (described above) containing the ctxB gene was then ligated to pJMK12 to yield pCVD622.2B. The relative position and orientation of the inserted genes is shown in Figure 19.

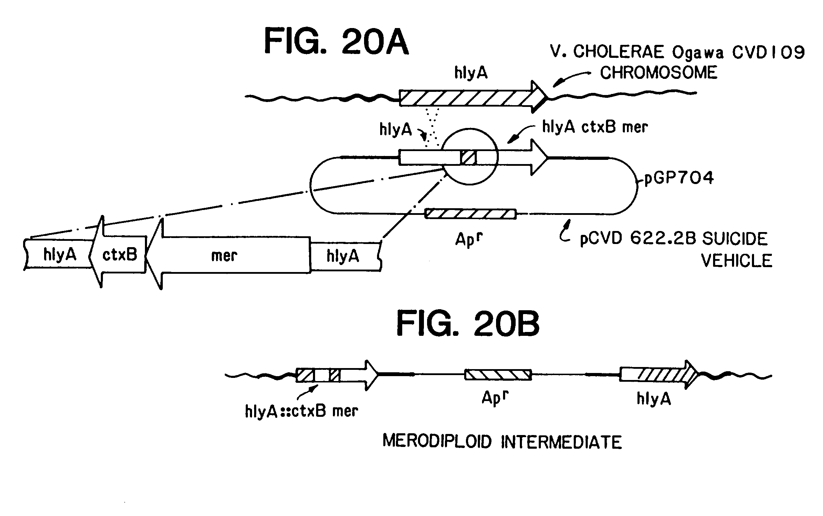

pCVD622.2B was then introduced into V. cholerae CVD109 by conjugation from an E. coli host strain. As described in Example 10, pGP704 cannot replicate extrachromosomally in V cholerae but can replicate in permissive E. coli strains. Since pCVD622.2B cannot replicate extrachromosomally in V. cholerae, selection of Ap^{r} colonies [pGP704 contains a gene encoding ampicillin resistance] yielded strains in which the entire pCVD622.2B plasmid, with the Ap^{r} marker, was homologously recombined into the chromosome at the site of the hylA gene. [It could not recombine into the ctx or zot sequences because CVD109 lacks these genes.] The result of this single cross-over (not double cross-over) event is termed a "cointegrate" structure or "merodiploiod" (these terms are used interchangeably) and is depicted in Figure 20B.

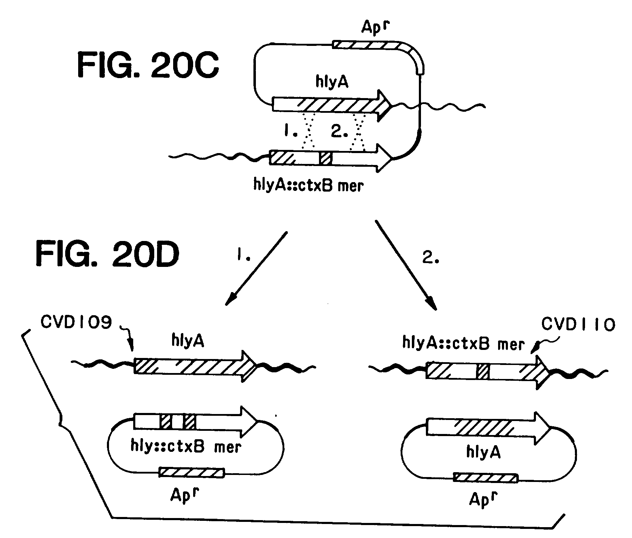

A second cross-over event can occur between the homologous hylA sequences flanking the integrated pCVD622.2B. This second cross-over event occurs spontaneously and is detected by selection of colonies which have lost the Ap^{r} phenotype. This second cross-over event can have one of two possible outcomes, depending upon the exact site of recombination. Both possible outcomes result in the loss of the pGP704 plasmid vector sequences and are depicted in Figures 20C and 20D. One outcome simply regenerates the original situation, i.e., a strain identical to CVD109 which lacks ctx, zot, and mer. The second outcome results in the lost of pGP704 sequences but the mer and ctx sequences contained within the hlyA sequences are retained. The two possible outcomes are readily distinguished by DNA hybridization using radiolabeled ctx sequences as a probe. To isolate the desired outcome, a culture of CVD109 containing the integrated pCVD622.2B was grown up in L-broth without added antibiotics. This culture was plated on non-selective L-agar plates and the resulting colonies were replicated onto Ap containing L-agar plates. Ap^{s} colonies were then hybridized to the ctx probe and colonies possessing ctx sequences were isolated. One such colony was designated V. cholerae CVD110. This strain was confirmed by DNA hybridization to contain ctx and mer sequences and to also lack pGP704 sequences and the 400 bp Hpa I fragment internal to the hlyA gene. V. cholerae CVD110 was also confirmed to produce the B subunit of cholera toxin by ELISA [Sack, D.A. et al. supra (1980)].

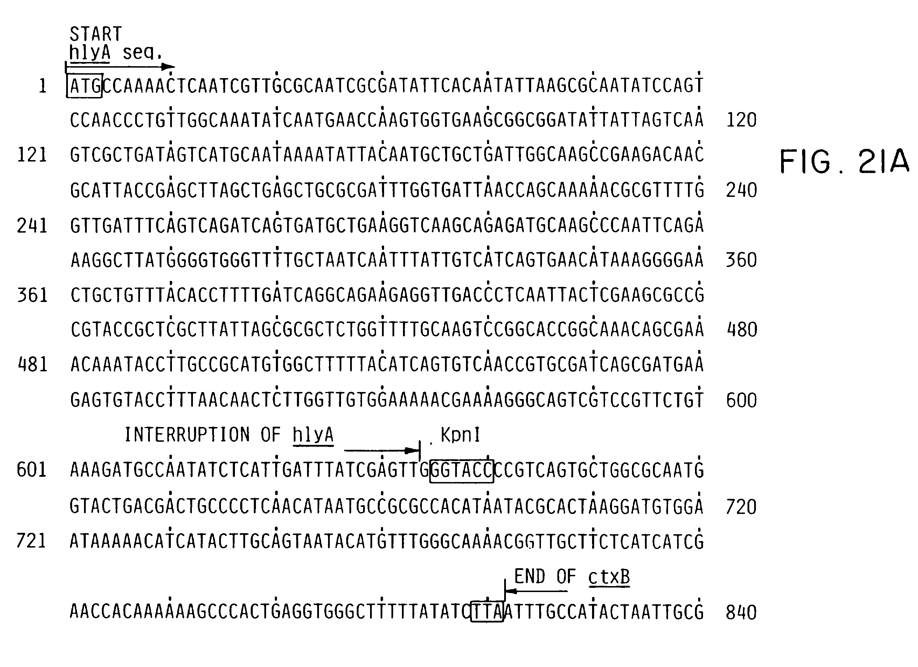

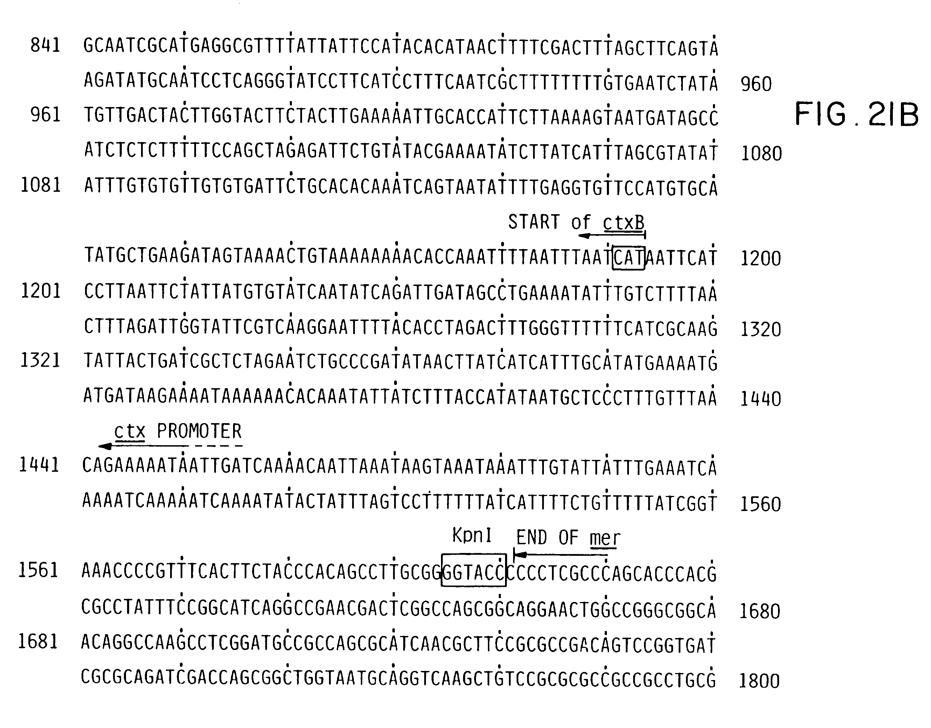

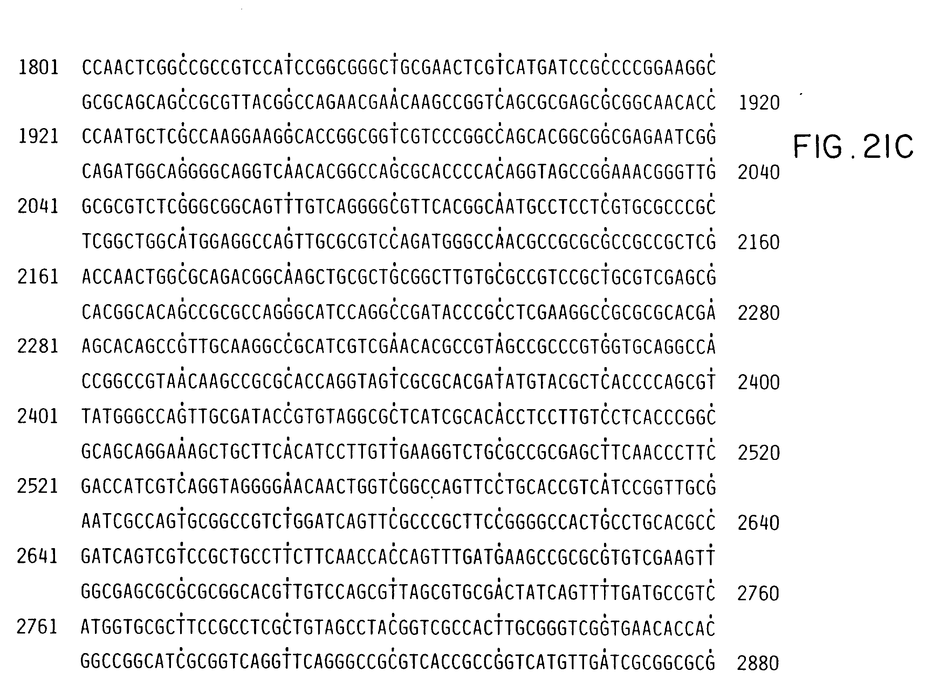

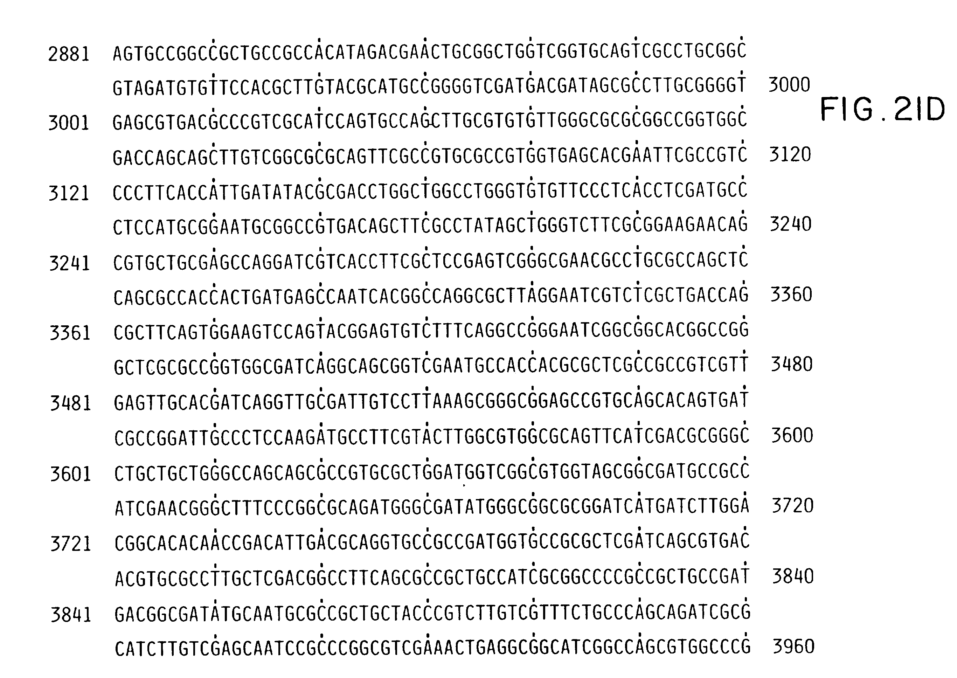

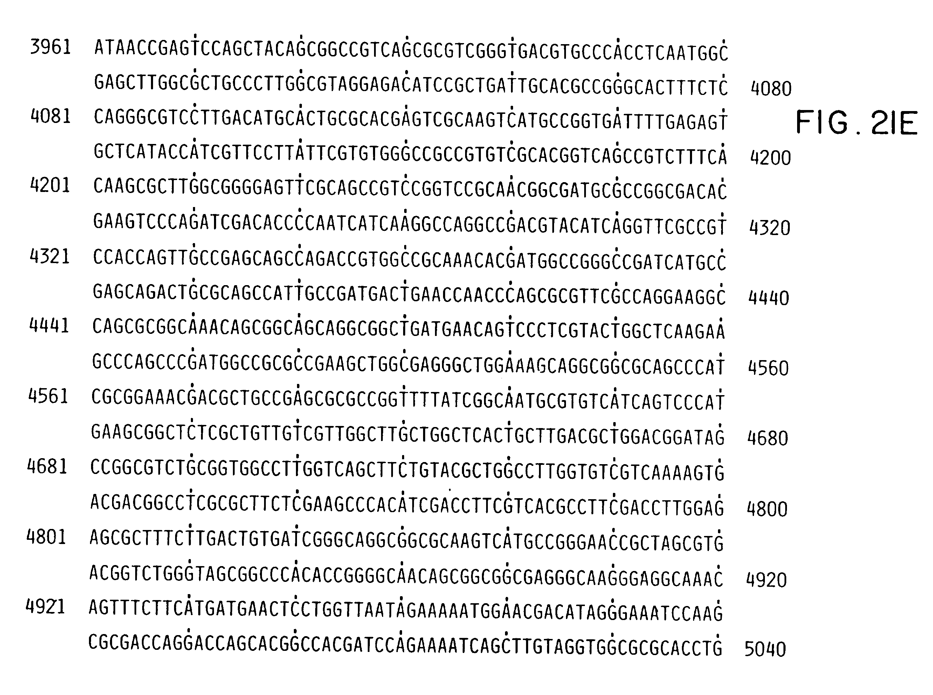

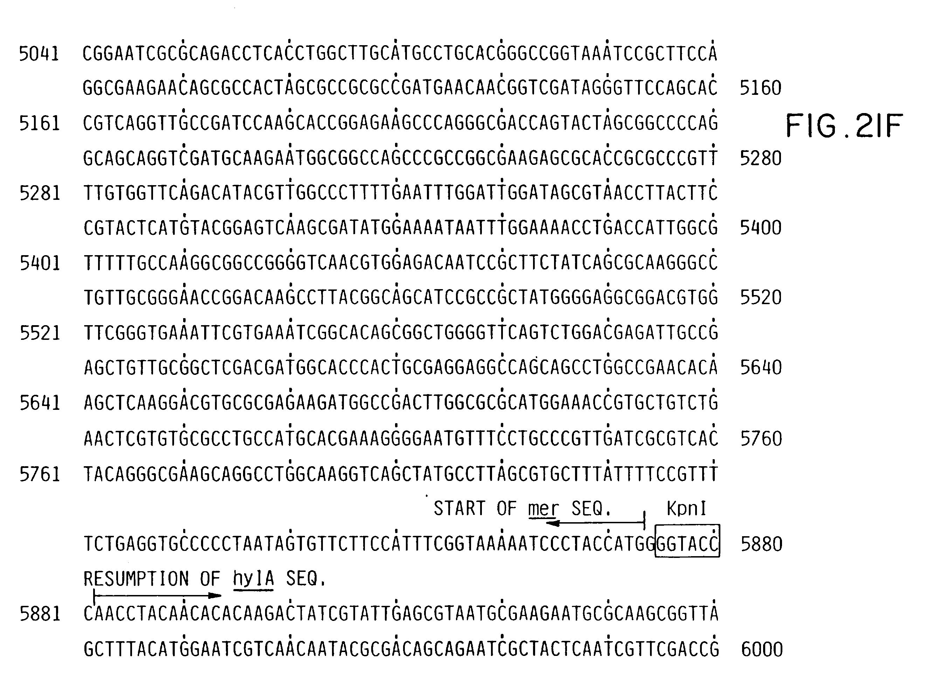

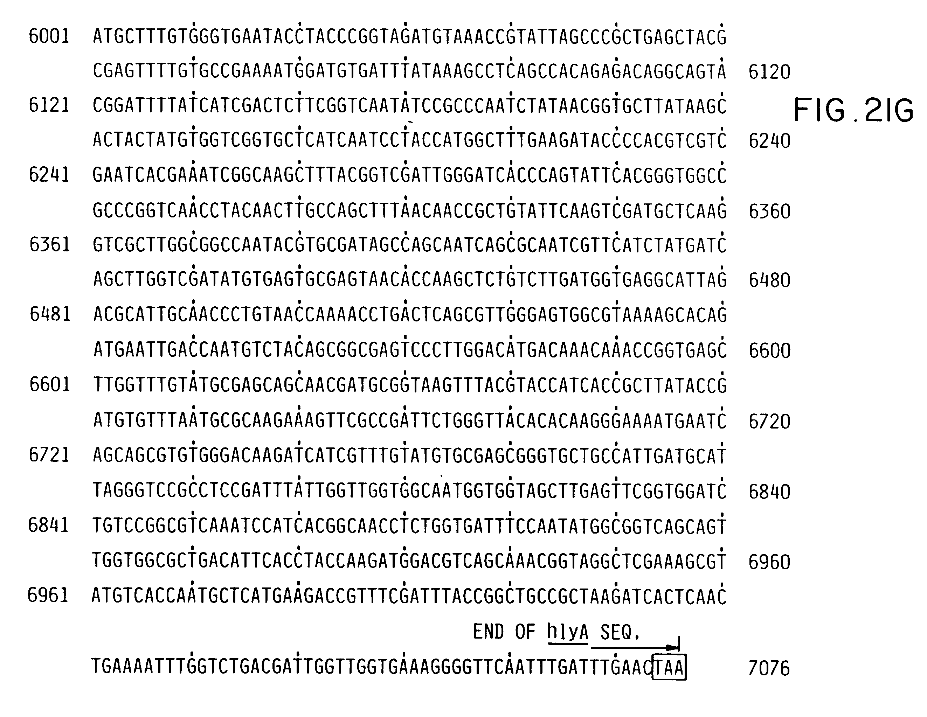

DNA sequence of inserted genes: The exact DNA sequences of the inserted ctx and mer genes are known from the literature. The exact site of the hlyA gene into which these genes were inserted is also known. Figure 21 presents the expected DNA sequence of these genes, showing the relative positions of each gene. The start and stop points of the different genes are indicated; direction of the arrows indicates direction of transcription of the gene. Only those sequences which are retained in the final construction are presented, e.g., ctxB is given but the portion of ctxA which was deleted in this construction is not shown.

While the invention has been described in connection with specific embodiments thereof, it will be understood that it is capable of further modification and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims (Claims for the following Contracting State(s): AT, BE, CH, DE, DK, FR, GB, IT, LI, LU, NL, SE)

1. A culture of *Vibrio cholerae* comprising a *Vibrio cholerae* strain of the Ogawa or Inaba serotype having a first fragment of DNA coding for *Vibrio cholerae* toxin or the A₁ subunit thereof deleted to confer avirulence and retaining the capacity to colonize the intestine of a host animal, and having a second fragment of DNA coding for zonula occludens toxin or a fragment thereof deleted to reduce residual side effects in said host animal.

2. The culture of *Vibrio cholerae* according to claim 1 wherein said first deleted DNA fragment codes for a portion of the *Vibrio cholerae* toxin.

3. The culture of *Vibrio cholerae* according to claims 1 or 2 wherein said first fragment of DNA comprises the A₁ subunit of the *ctx* gene.

4. The culture of *Vibrio cholerae* according to any one of claims 1 to 3 wherein said culture is useful for vaccination against cholera.

5. A culture of *Vibrio cholerae* comprising a *Vibrio cholerae* of the Ogawa or Inaba serotype having a region of the chromosomal DNA coding for cholera toxin and zonula occludens toxin deleted to confer avirulence and retain capacity to colonize the intestine of a host animal and to reduce residual side effects in said host animal.

6. A method of isolating deletion mutants of *Vibrio cholerae* having a region of the chromosomal DNA coding for cholera toxin, or a part thereof, and zonula occludens toxin deleted comprising:
(a) obtaining a plasmid comprising *Vibrio cholerae* DNA sequences coding for cholera toxin and zonula occludens toxin and a gene for a selectable marker of foreign origin, wherein said plasmid is incapable of replicating extrachromosomally in *Vibrio cholerae;*
(b) mating a microorganism carrying said plasmid with a virulent strain of *Vibrio cholerae* containing said sequences coding for cholera toxin and zonula occludens toxin inserted between flanking copies of a second sequence which promotes detectable in vivo recombination;
(c) selecting for *Vibrio cholerae* expressing said selectable marker;
(d) growing the selected product of (c) in the absence of a selective agent;
(e) selecting for *Vibrio cholerae* which no longer express the selective marker.

7. A method of isolating deletion mutants of *Vibrio cholerae* according to claim 6, wherein said second sequence comprises an approximately 2700 base pair repetitive sequence (RS1) element.

8. A method according to claim 6, wherein said plasmid is pCVD51 (ATCC No. 68,335).

9. A method according to claim 6, wherein said virulent strain is E7946 (ATCC No. 55.056).

10. A method of isolating deletion mutants of *Vibrio cholerae* having a deletion in the *ctx* gene, or a part thereof, and a deletion the *zot* gene, comprising:
(a) obtaining a first plasmid comprising *Vibrio cholerae* flanking sequences of one or more deleted DNA fragments and a gene for a selectable marker of foreign origin ligated to said flanking sequences to substitute for and to be in place of said deleted fragment, wherein said flanking sequences promote detectable *in vivo* recombination;
(b) mating a virulent strain of *Vibrio cholerae* with a first microorganism carrying the first plasmid;
(c) selecting for *Vibrio cholerae* expressing the first selectable marker;
(d) mating the selected product of step (c) with a second microorganism carrying a second plasmid with a second selectable marker, said second plasmid being incompatible with the first plasmid;
(e) selecting for *Vibrio cholerae* expressing both the first selectable marker and the second selectable marker;
(f) obtaining a third plasmid comprising *Vibrio cholerae* flanking sequences of one or more deleted DNA fragments homologous to those described in step (a) but differing in the absence of a selectable marker of foreign origin;
(g) mating the selected product of step (e) with a third microorganism carrying the third plasmid; and
(h) selecting for *Vibrio cholerae* which no longer expresses the first selectable marker.

11. A method of isolating deletion mutants of *Vibrio cholerae* according to any one of claims 6 to 10, wherein said deletion in the *ctx* gene is defined by *XbaI* and *ClaI* restriction endonuclease sites and said deletion in the *zot* gene is defined by *StuI* and *AccI* restriction endonuclease sites.

12. The culture of *Vibrio cholerae* according to any one of claims 1 to 5, wherein the first deleted DNA fragment is deleted of the A subunit and the B subunit genes of *Vibrio cholerae* toxin.

13. The culture of *Vibrio cholerae* according to claim 12, wherein said *Vibrio cholerae* strain has a mercury resistance gene and a DNA coding for the B subunit of *Vibrio cholerae* toxin inserted into the chromosome of *Vibrio cholerae.*

14. The culture of *Vibrio cholerae* according to claim 13, wherein said mercury resistance gene and DNA coding for the B subunit of *Vibrio cholerae* toxin are inserted at the site of a hemolysin gene in the chromosome of *Vibrio cholerae.*

15. The culture of *Vibrio cholerae* according to claim 14, wherein said culture comprises *Vibrio cholerae* CVD 110 (ATCC No. 55,188).

16. A method of isolating deletion mutants of *Vibrio cholerae* having a region of chromosomal DNA coding for cholera toxin and zonula occludens toxin deleted, and having inserted a mercury resistance gene and DNA coding for B subunit of *Vibrio cholerae* toxin comprising:
(a) obtaining a plasmid comprising *Vibrio cholerae* sequences coding for cholera toxin and zonula occludens toxin and a gene for a selectable marker of foreign origin, wherein said plasmid is incapable of replicating extrachromosomally in *Vibrio cholerae;*
(b) mating a microorganism carrying said plasmid with a virulent strain of *Vibrio cholerae* containing said sequences coding for cholera toxin and zonula occludens toxin inserted between flanking copies of a second sequence which promotes detectable *in vivo* recombination;
(c) selecting for *Vibrio cholerae* expressing said selectable marker;
(d) growing the selected product of step (c) in the absence of a selective agent;
(e) selecting for *Vibrio cholerae* which no longer express the selective marker;
(f) obtaining a second plasmid comprising a mercury resistance gene and DNA coding for the B subunit of *Vibrio cholerae* toxin and a gene for a second selectable marker of foreign origin, wherein said second plasmid is incapable of replicating extrachomosomally in *Vibrio cholerae,* and where sequences which promote detectable *in vivo* recombination flank said mercury resistance gene and DNA coding for the B subunit of *Vibrio cholerae* toxin;
(g) mating a microorganism carrying said second plasmid with said *Vibrio cholerae* recited in step (e);
(h) selecting for *Vibrio cholerae* expressing said second selectable marker;
(i) growing the selected product of step (h) in the absence of a second selective agent;
(j) selecting for *Vibrio cholerae* which no longer express said second selective marker; and
(k) screening said *Vibrio cholerae* recited in step (j) for *Vibrio cholerae* that have a mercury resistance gene and DNA coding for the B subunit of *Vibrio cholerae* toxin, and have a region of chromosomal DNA coding for cholera toxin and zonula occludens toxin deleted.

17. The method of isolating deletion mutants of *Vibrio cholerae* according to claim 16, wherein said second plasmid is pCVD662.2B (ATCC No. 68,630).

18. The method of isolating deletion mutants of *Vibrio cholerae* according to claims 16 or 17, wherein said flanking sequences comprise hemolysin gene sequences.

19. The method of isolating deletion mutants of *Vibrio cholerae* according to claim 16, wherein said *Vibrio cholerae* recited in step (e) is *Vibrio cholerae* CVD 109 (ATCC No. 55,057).

20. The method of isolating deletion mutants of *Vibrio cholerae* according to any one of claims 16 to 19, wherein said *Vibrio cholerae* selected in step (j) is *Vibrio cholerae* CVD 110 (ATCC No. 55,188).

21. The method of isolating deletion mutants of *Vibrio cholerae* according to any one of claims 16 to 19, wherein said selectable marker and said second selectable marker are ampicillin resistance.

22. The method of isolating deletion mutants of *Vibrio cholerae* according to any one of claims 16 to 19, wherein said selectable marker is ampicillin resistance and said second selectable marker is chloramphenicol resistance.

23. *Vibrio cholerae* CVD 110 (ATCC No. 55,188).

24. A vaccine for protecting against the symptoms of cholera comprising a *Vibrio cholerae* strain of the Ogawa or Inaba serotype having a first fragment of DNA coding for *Vibrio cholerae* toxin or the A₁ subunit thereof deleted to confer avirulence and retaining the capacity to colonize the intestine of a host animal and having a second fragment of DNA coding for zonula occludens toxin or a fragment thereof deleted.

25. The vaccine according to claim 24, wherein said *Vibrio cholerae* is *Vibrio cholerae* CVD 110 (ATCC No. 55,188).

## Claims (Claims for the following Contracting State(s): GR, ES)

1. A culture of *Vibrio cholerae* comprising a *Vibrio cholerae* strain of the Ogawa or Inaba serotype having a first fragment of DNA coding for *Vibrio cholerae* toxin or the A₁ subunit thereof deleted to confer avirulence and retaining the capacity to colonize the intestine of a host animal, and having a second fragment of DNA coding for zonula occludens toxin or a fragment thereof deleted to reduce residual side effects in said host animal.

2. The culture of *Vibrio cholerae* according to claim 1 wherein said first deleted DNA fragment codes for a portion of the *Vibrio cholerae* toxin.

3. The culture of *Vibrio cholerae* according to claims 1 or 2 wherein said first fragment of DNA comprises the A₁ subunit of the *ctx* gene.

4. The culture of *Vibrio cholerae* according to any one of claims 1 to 3 wherein said culture is useful for vaccination against cholera.

5. A culture of *Vibrio cholerae* comprising a *Vibrio cholerae* of the Ogawa or Inaba serotype having a region of the chromosomal DNA coding for cholera toxin and zonula occludens toxin deleted to confer avirulence and retain capacity to colonize the intestine of a host animal and to reduce residual side effects in said host animal.

6. A method of isolating deletion mutants of *Vibrio cholerae* having a region of the chromosomal DNA coding for cholera toxin, or a part thereof, and zonula occludens toxin deleted comprising:
(a) obtaining a plasmid comprising *Vibrio cholerae* DNA sequences coding for cholera toxin and zonula occludens toxin and a gene for a selectable marker of foreign origin, wherein said plasmid is incapable of replicating extrachromosomally in *Vibrio cholerae;*
(b) mating a microorganism carrying said plasmid with a virulent strain of *Vibrio cholerae* containing said sequences coding for cholera toxin and zonula occludens toxin inserted between flanking copies of a second sequence which promotes detectable in vivo recombination;
(c) selecting for *Vibrio cholerae* expressing said selectable marker;
(d) growing the selected product of (c) in the absence of a selective agent;
(e) selecting for *Vibrio cholerae* which no longer express the selective marker.

7. A method of isolating deletion mutants of *Vibrio cholerae* according to claim 6, wherein said second sequence comprises an approximately 2700 base pair repetitive sequence (RS1) element.

8. A method according to claim 6, wherein said plasmid is pCVD51 (ATCC No. 68,335).

9. A method according to claim 6, wherein said virulent strain is E7946 (ATCC No. 55.056).

10. A method of isolating deletion mutants of *Vibrio cholerae* having a deletion in the *ctx* gene, or a part thereof, and a deletion the *zot* gene, comprising:
(a) obtaining a first plasmid comprising *Vibrio cholerae* flanking sequences of one or more deleted DNA fragments and a gene for a selectable marker of foreign origin ligated to said flanking sequences to substitute for and to be in place of said deleted fragment, wherein said flanking sequences promote detectable *in vivo* recombination;
(b) mating a virulent strain of *Vibrio cholerae* with a first microorganism carrying the first plasmid;
(c) selecting for *Vibrio cholerae* expressing the first selectable marker;
(d) mating the selected product of step (c) with a second microorganism carrying a second plasmid with a second selectable marker, said second plasmid being incompatible with the first plasmid;
(e) selecting for *Vibrio cholerae* expressing both the first selectable marker and the second selectable marker;
(f) obtaining a third plasmid comprising *Vibrio cholerae* flanking sequences of one or more deleted DNA fragments homologous to those described in step (a) but differing in the absence of a selectable marker of foreign origin;
(g) mating the selected product of step (e) with a third microorganism carrying the third plasmid; and
(h) selecting for *Vibrio cholerae* which no longer expresses the first selectable marker.

11. A method of isolating deletion mutants of *Vibrio cholerae* according to any one of claims 6 to 10, wherein said deletion in the *ctx* gene is defined by *XbaI* and *ClaI* restriction endonuclease sites and said deletion in the *zot* gene is defined by *StuI* and *AccI* restriction endonuclease sites.

12. The culture of *Vibrio cholerae* according to any one of claims 1 to 5, wherein the first deleted DNA fragment is deleted of the A subunit and the B subunit genes of *Vibrio cholerae* toxin.

13. The culture of *Vibrio cholerae* according to claim 12, wherein said *Vibrio cholerae* strain has a mercury resistance gene and a DNA coding for the B subunit of *Vibrio cholerae* toxin inserted into the chromosome of *Vibrio cholerae.*

14. The culture of *Vibrio cholerae* according to claim 13, wherein said mercury resistance gene and DNA coding for the B subunit of *Vibrio cholerae* toxin are inserted at the site of a hemolysin gene in the chromosome of *Vibrio cholerae.*

15. The culture of *Vibrio cholerae* according to claim 14, wherein said culture comprises *Vibrio cholerae* CVD 110 (ATCC No. 55,188).

16. A method of isolating deletion mutants of *Vibrio cholerae* having a region of chromosomal DNA coding for cholera toxin and zonula occludens toxin deleted, and having inserted a mercury resistance gene and DNA coding for B subunit of *Vibrio cholerae* toxin comprising:
(a) obtaining a plasmid comprising *Vibrio cholerae* sequences coding for cholera toxin and zonula occludens toxin and a gene for a selectable marker of foreign origin, wherein said plasmid is incapable of replicating extrachromosomally in *Vibrio cholerae;*
(b) mating a microorganism carrying said plasmid with a virulent strain of *Vibrio cholerae* containing said sequences coding for cholera toxin and zonula occludens toxin inserted between flanking copies of a second sequence which promotes detectable *in vivo* recombination;
(c) selecting for *Vibrio cholerae* expressing said selectable marker;
(d) growing the selected product of step (c) in the absence of a selective agent;
(e) selecting for *Vibrio cholerae* which no longer express the selective marker;
(f) obtaining a second plasmid comprising a mercury resistance gene and DNA coding for the B subunit of *Vibrio cholerae* toxin and a gene for a second selectable marker of foreign origin, wherein said second plasmid is incapable of replicating extrachomosomally in *Vibrio cholerae,* and where sequences which promote detectable *in vivo* recombination flank said mercury resistance gene and DNA coding for the B subunit of *Vibrio cholerae* toxin;
(g) mating a microorganism carrying said second plasmid with said *Vibrio cholerae* recited in step (e);
(h) selecting for *Vibrio cholerae* expressing said second selectable marker;
(i) growing the selected product of step (h) in the absence of a second selective agent;
(j) selecting for *Vibrio cholerae* which no longer express said second selective marker; and
(k) screening said *Vibrio cholerae* recited in step (j) for *Vibrio cholerae* that have a mercury resistance gene and DNA coding for the B subunit of *Vibrio cholerae* toxin, and have a region of chromosomal DNA coding for cholera toxin and zonula occludens toxin deleted.

17. The method of isolating deletion mutants of *Vibrio cholerae* according to claim 16, wherein said second plasmid is pCVD662.2B (ATCC No. 68,630).

18. The method of isolating deletion mutants of *Vibrio cholerae* according to claims 16 or 17, wherein said flanking sequences comprise hemolysin gene sequences.

19. The method of isolating deletion mutants of *Vibrio cholerae* according to claim 16, wherein said *Vibrio cholerae* recited in step (e) is *Vibrio cholerae* CVD 109 (ATCC No. 55,057).

20. The method of isolating deletion mutants of *Vibrio cholerae* according to any one of claims 16 to 19, wherein said *Vibrio cholerae* selected in step (j) is *Vibrio cholerae* CVD 110 (ATCC No. 55,188).

21. The method of isolating deletion mutants of *Vibrio cholerae* according to any one of claims 16 to 19, wherein said selectable marker and said second selectable marker are ampicillin resistance.

22. The method of isolating deletion mutants of *Vibrio cholerae* according to any one of claims 16 to 19, wherein said selectable marker is ampicillin resistance and said second selectable marker is chloramphenicol resistance.

23. *Vibrio cholerae* CVD 110 (ATCC No. 55,188).

24. A process for preparing a vaccine for protecting against the symptoms of cholerae, comprising combining a *Vibrio cholerae* strain of the Ogawa or Inaba serotype, said strain having a first fragment of DNA coding for *Vibrio cholerae* toxin or the A₁ subunit thereof deleted to confer avirulence and retaining the capacity to colonize the intestine of a host animal and having a second fragment of DNA coding for zonula occludens toxin or a fragment thereof deleted, and a pharmaceutically acceptable diluent.

25. The method according to claim 24, wherein said Vibrio cholerae is *Vibrio cholerae* CVD110(ATTC No.55188).

26. The process according to claim 24, wherein said first deleted DNA fragment codes for a portion of the *Vibrio cholerae* toxin.

27. The process according to claim 24, wherein said first fragment of DNA comprises the A₁ subunit of the *ctx* gene.

28. The process according to claim 24, wherein the first deleted DNA fragment is deleted of the A subunit and the B subunit genes of *Vibrio cholerae* toxin.

29. The process according to claim 28, wherein said *Vibrio cholerae* strain has a mercury resistance gene and a DNA coding for the B subunit of the *Vibrio cholerae.*

30. The process according to claim 29, wherein said mercury resistance gene and DNA coding for the B subunit of the *Vibrio cholerae* toxin are inserted at the site of a hemolysin gene in the chromosome of *Vibrio cholerae.*

## Patentansprüche (Patentansprüche für folgende(n) Vertragsstaat(en): AT, BE, CH, DE, DK, FR, GB, IT, LI, LU, NL, SE)

1. Vibrio cholerae-Kultur, umfassend einen Vibrio cholerae-Stamm vom Ogawa- oder Inaba-Serotyp, bei dem ein erstes DNA-Fragment deletiert ist, das für das Vibrio cholerae-Toxin oder die A₁-Untereinheit davon kodiert, um Avirulenz zu verleihen, wobei der Vibrio cholerae-Stamm die Fähigkeit zur Besiedelung des Darmes eines Wirtstieres beibehält, und bei dem ein zweites DNA-Fragment, das für das Zonula-occludens-Toxin oder ein Fragment davon kodiert, deletiert ist, um die restlichen Nebenwirkungen in dem Wirtstier zu verringern.

2. Vibrio cholerae-Kultur gemäß Anspruch 1, bei der das erste deletierte DNA-Fragment für einen Teil des Vibrio cholerae-Toxines kodiert.

3. Vibrio cholerae-Kultur nach Anspruch 1 oder 2, bei der das erste DNA-Fragment die A₁-Untereinheit des ctx-Genes umfaßt.

4. Vibrio cholerae-Kultur nach einem der Ansprüche 1 bis 3, wobei die Kultur für eine Impfung gegen Cholera nützlich ist.

5. Vibrio cholerae-Kultur, umfassend einen Vibrio cholerae des Ogawa- oder Inaba-Serotyps, bei dem ein Bereich der chromosomalen DNA deletiert ist, der für das Cholera-Toxin und das Zonula-occludens-Toxin kodiert, um Avirulenz zu verleihen und die Fähigkeit zur Besiedelung des Darmes eines Wirtstieres beizubehalten und um die restlichen Nebenwirkungen in dem Wirtstier zu verringern.

6. Verfahren zum Isolieren von Deletionsmutanten von Vibrio cholerae, bei denen ein Bereich der chromosomalen DNA, der für Cholera-Toxin oder einen Teil davon und Zonula-occludens-Toxin kodiert, deletiert ist, umfassend
a) das Erhalten eines Plasmides, das Vibrio cholerae-DNA-Sequenzen, die für Cholera-Toxin und Zonula-occludens-Toxin kodieren, und ein Gen für einen selektierbaren Marker fremden Ursprungs umfaßt, wobei das Plasmid in Vibrio cholerae nicht extrachromosomal replizieren kann;
b) das Vereinigen (mating) eines Mikroorganismus, der das Plasmid trägt, mit einem virulenten Vibrio cholerae-Stamm, der die für Cholera-Toxin und Zonula-occludens-Toxin kodierenden Sequenzen zwischen flankierenden Kopien einer zweiten Sequenz insertiert enthält, was eine nachweisbare in vivo-Rekombination fördert;
c) das Auswählen von Vibrio cholerae, die den selektierbaren Marker exprimieren;
d) das Wachsenlassen des ausgewählten Produktes von (c) in Abwesenheit eines selektiven Mittel;
e) das Auswählen von Vibrio cholerae, die den selektiven Marker nicht länger exprimieren.

7. Verfahren zum Isolieren von Deletionsmutanten von Vibrio cholerae gemäß Anspruch 6, wobei die zweite Sequenz ein ungefähr 2700 Basenpaare langes repetitives Sequenzelement (RS1) umfaßt.

8. Verfahren nach Anspruch 6, wobei das Plasmid pCVD51 (ATCC Nr. 68,335) ist.

9. Verfahren nach Anspruch 6, wobei der virulente Stamm E7946 (ATCC Nr. 55.056) ist.

10. Verfahren zum Isolieren von Deletionsmutanten von Vibrio cholerae, die eine Deletion im ctx-Gen oder einem Teil davon und eine Deletion im zot-Gen haben, umfassend:
a) das Erhalten eines ersten Plasmides, das flankierende Vibrio cholerae-Sequenzen eines oder mehrerer deletierter DNA-Fragmente umfaßt und ein Gen für einen selektierbaren Marker fremden Ursprungs, der an die flankierenden Sequenzen ligiert ist, um das deletierte Fragment zu substituieren und anstelle des deletierten Fragmentes zu setzen, wobei die flankierenden Sequenzen die nachweisbare in vivo-Rekombination fördern;
b) das Vereinigen eines virulenten Vibrio cholerae-Stammes mit einem ersten Mikroorganismus, der das erste Plasmid trägt;
c) das Auswählen von Vibrio cholerae, die den ersten selektierbaren Marker exprimieren;
d) das Vereinigen des ausgewählten Produktes von Schritt c) mit einem zweiten Mikroorganismus, der ein zweites Plasmid mit einem zweiten selektierbaren Marker trägt, wobei das zweite Plasmid mit dem ersten Plasmid inkompatibel ist;
e) das Auswählen von Vibrio cholerae, die sowohl den ersten selektierbaren Marker als auch den zweiten selektierbaren Marker exprimieren;
f) das Erhalten eines dritten Plasmides, das flankierende Vibrio cholerae-Sequenzen von einem oder mehreren deletierten DNA-Fragmenten umfaßt, die den in Schritt a) beschriebenen homolog sind, sich jedoch durch die Abwesenheit eines selektierbaren Marker fremden Ursprungs unterscheiden;
g) das Vereinigen des ausgewählten Produktes von Schritt e) mit einem dritten Mikroorganismus, der das dritte Plasmid trägt; und
h) das Auswählen von Vibrio cholerae, die den ersten selektierbaren Marker nicht länger exprimieren.

11. Verfahren zum Isolieren von Deletionsmutanten von Vibrio cholerae gemäß einem der Ansprüche 6 bis 10, wobei die Deletion in dem ctx-Gen durch XbaI- und ClaI-Restriktionsendonukleaseschnittstellen definiert ist und die Deletion im zot-Gen durch StuI- und AccI-Restriktionsendonukleaseschnittstellen definiert ist.

12. Vibrio cholerae-Kultur gemäß einem der Ansprüche 1 bis 5, bei der das erste deletierte DNA-Fragment aus den Genen der A- und der B-Untereinheit des Vibrio cholerae-Toxines deletiert ist.

13. Vibrio cholerae-Kultur gemäß Anspruch 12, worin der Vibrio cholerae-Stamm ein Quecksilberresistenzgen hat und eine für die B-Untereinheit des Vibrio cholerae-Toxins kodierende DNA in das Chromosom des Vibrio cholerae insertiert ist.

14. Vibrio cholerae-Kultur nach Anspruch 13, wobei das Quecksilberresistenzgen und die für die B-Untereinheit von Vibrio cholerae-Toxin kodierende DNA an der Stelle eines Hämolysingenes im Vibrio cholerae-Chromosom insertiert sind.

15. Vibrio cholerae-Kultur nach Anspruch 14, wobei die Kultur Vibrio cholerae CVD 110 (ATTC Nr. 55,188) umfaßt.

16. Verfahren zum Isolieren von Deletionsmutanten von Vibrio cholerae, bei denen ein Bereich chromosomaler DNA, der für Cholera-Toxin und Zonula-occludens-Toxin kodiert, deletiert ist, und bei dem ein Quecksilberresistenzgen und eine für die B-Untereinheit von Vibrio cholerae-Toxin kodierende DNA insertiert ist, umfassend:
a) das Erhalten eines Plasmides, das Vibrio cholerae-DNA-Sequenzen, die für Cholera-Toxin und Zonula-occludens-Toxin kodieren, und ein Gen für einen selektierbaren Marker fremden Ursprungs umfaßt, wobei das Plasmid in Vibrio cholerae nicht extrachromosomal replizieren kann;
b) das Vereinen eines Mikroorganismus, der das Plasmid trägt, mit einem virulenten Vibrio cholerae-Stamm, der die für Cholera-Toxin und Zonula-occludens-Toxin kodierenden Sequenzen zwischen flankierenden Kopien einer zweiten Sequenz insertiert enthält, was die nachweisbare in vivo-Rekombination fördert;
c) das Auswählen von Vibrio cholerae, die den selektierbaren Marker exprimieren;
d) das Wachsenlassen des ausgewählten Produktes von Schritt (c) in Abwesenheit eines selektiven Agenzes;
e) das Auswählen von Vibrio cholerae, die den selektiven Marker nicht länger exprimieren;
f) das Erhalten eines zweiten Plasmides, das ein Quecksilberresistenzgen und eine für die B-Untereinheit des Vibrio cholerae-Toxines kodierende DNA und ein Gen für einen zweiten selektierbaren Marker fremden Ursprungs enthält, wobei das zweite Plasmid in Vibrio cholerae nicht extrachromosomal replizieren kann, und wo Sequenzen, die nachweisbare in vivo-Rekombination fördern, das Quecksilberresistenzgen und die für die B-Untereinheit des Vibrio cholerae-Toxins kodierende DNA flankieren;
g) das Vereinen eines Mikroorganismus, der das zweite Plasmid trägt, mit Vibrio cholerae gemäß Schritt e);
h) das Auswählen von Vibrio cholerae, die den zweiten selektierbaren Marker exprimieren;
i) das Wachsenlassen des ausgewählten Produktes von Schritt h) in Abwesenheit eines zweiten selektiven Markers;
j) das Auswählen von Vibrio cholerae, die den zweiten selektiven Marker nicht länger exprimieren; und
k) das Durchmustern von in Schritt j) erwähnten Vibrio cholerae auf Vibrio cholerae, die ein Quecksilberresistenzgen und für die B-Untereinheit von Vibrio cholerae-Toxin kodierende DNA enthalten und bei denen ein Bereich chromosomaler DNA, der für Cholera-Toxin und Zonula-occludens-Toxin kodiert, deletiert ist.

17. Verfahren zum Isolieren von Deletionsmutanten von Vibrio cholerae gemäß Anspruch 16, wobei das zweite Plasmid pCVD662.2B (ATTC NR. 68,630) ist.

18. Verfahren zum Isolieren von Deletionsmutanten von Vibrio cholerae gemäß Anspruch 16 oder 17, worin die flankierenden Sequenzen Hemolysingensequenzen enthalten.

19. Verfahren zum Isolieren von Deletionsmutanten von Vibrio cholerae gemäß Anspruch 16, wobei der in Schritt e) erwähnte Vibrio cholerae Vibrio cholerae CVD 109 (ATCC Nr. 55,057) ist.

20. Verfahren zum Isolieren von Deletionsmutanten von Vibrio cholerae gemäß einem der Ansprüche 16 bis 19, worin der in Schritt j) ausgewählte Vibrio cholerae Vibrio cholerae CVD 110 (ATCC Nr. 55,188) ist.

21. Verfahren zum Isolieren von Deletionsmutanten von Vibrio cholerae gemäß einem der Ansprüche 16 bis 19, worin der selektierbare Marker und der zweite selektierbare Marker Ampicillinresistenz sind.

22. Verfahren zum Isolieren von Deletionsmutanten von Vibrio cholerae gemäß einem der Ansprüche 16 bis 19, worin der selektierbare Marker Ampicillinresistenz und der zweite selektierbare Marker Chloramphenicolresistenz ist.

23. Vibrio cholerae CVD 110 (ATCC Nr. 55,188).

24. Impfstoff zum Schützen gegen die Symptome von Cholera, umfassend einen Vibrio cholerae-Stamm des Ogawa- oder Inaba-Serotyps, bei dem ein erstes DNA-Fragment, das für das Vibrio cholerae-Toxin oder die A₁-Untereinheit davon kodiert, deletiert ist, um Avirulenz zu verleihen, und der die Fähigkeit zur Besiedelung des Darmes eines Wirtstieres beibehält, und bei dem ein zweites DNA-Fragment, das für das Zonula-occludens-Toxin oder ein Fragment davon kodiert, deletiert ist.

25. Impfstoff nach Anspruch 24, worin der Vibrio cholerae Vibrio cholerae CVD 110 (ATCC Nr. 55, 188) ist.

## Patentansprüche (Patentansprüche für folgende(n) Vertragsstaat(en): ES, GR)

1. Vibrio cholerae-Kultur, umfassend einen Vibrio cholerae-Stamm vom Ogawa- oder Inaba-Serotyp, bei dem ein erstes DNA-Fragment deletiert ist, das für das Vibrio cholerae-Toxin oder die A₁-Untereinheit davon kodiert, um Avirulenz zu verleihen, wobei der Vibrio cholerae-Stamm die Fähigkeit zur Besiedelung des Darmes eines Wirtstieres beibehält, und bei dem ein zweites DNA-Fragment, das für das Zonula-occludens-Toxin oder ein Fragment davon kodiert, deletiert ist, um die restlichen Nebenwirkungen in dem Wirtstier zu verringern.

2. Vibrio cholerae-Kultur gemäß Anspruch 1, bei der das erste deletierte DNA-Fragment für einen Teil des Vibrio cholerae-Toxines kodiert.

3. Vibrio cholerae-Kultur nach Anspruch 1 oder 2, bei der das erste DNA-Fragment die A₁-Untereinheit des ctx-Genes umfaßt.

4. Vibrio cholerae-Kultur nach einem der Ansprüche 1 bis 3, wobei die Kultur für eine Impfung gegen Cholera nützlich ist.

5. Vibrio cholerae-Kultur, umfassend einen Vibrio cholerae des Ogawa- oder Inaba-Serotyps, bei dem ein Bereich der chromosomalen DNA deletiert ist, der für das Cholera-Toxin und das Zonula-occludens-Toxin kodiert, um Avirulenz zu verleihen und die Fähigkeit zur Besiedelung des Darmes eines Wirtstieres beizubehalten und um die restlichen Nebenwirkungen in dem Wirtstier zu verringern.

6. Verfahren zum Isolieren von Deletionsmutanten von Vibrio cholerae, bei denen ein Bereich der chromosomalen DNA, der für Cholera-Toxin oder einen Teil davon und Zonula-occludens-Toxin kodiert, deletiert ist, umfassend
a) das Erhalten eines Plasmides, das Vibrio cholerae-DNA-Sequenzen, die für Cholera-Toxin und Zonula-occludens-Toxin kodieren, und ein Gen für einen selektierbaren Marker fremden Ursprungs umfaßt, wobei das Plasmid in Vibrio cholerae nicht extrachromosomal replizieren kann;
b) das Vereinigen (mating) eines Mikroorganismus, der das Plasmid trägt, mit einem virulenten Vibrio cholerae-Stamm, der die für Cholera-Toxin und Zonula-occludens-Toxin kodierenden Sequenzen zwischen flankierenden Kopien einer zweiten Sequenz insertiert enthält, was eine nachweisbare in vivo-Rekombination fördert;
c) das Auswählen von Vibrio cholerae, die den selektierbaren Marker exprimieren;
d) das Wachsenlassen des ausgewählten Produktes von (c) in Abwesenheit eines selektiven Mittel;
e) das Auswählen von Vibrio cholerae, die den selektiven Marker nicht länger exprimieren.

7. Verfahren zum Isolieren von Deletionsmutanten von Vibrio cholerae gemäß Anspruch 6, wobei die zweite Sequenz ein ungefähr 2700 Basenpaare langes repetitives Sequenzelement (RS1) umfaßt.

8. Verfahren nach Anspruch 6, wobei das Plasmid pCVD51 (ATCC Nr. 68,335) ist.

9. Verfahren nach Anspruch 6, wobei der virulente Stamm E7946 (ATCC Nr. 55.056) ist.

10. Verfahren zum Isolieren von Deletionsmutanten von Vibrio cholerae, die eine Deletion im ctx-Gen oder einem Teil davon und eine Deletion im zot-Gen haben, umfassend:
a) das Erhalten eines ersten Plasmides, das flankierende Vibrio cholerae-Sequenzen eines oder mehrerer deletierter DNA-Fragmente umfaßt und ein Gen für einen selektierbaren Marker fremden Ursprungs, der an die flankierenden Sequenzen ligiert ist, um das deletierte Fragment zu substituieren und anstelle des deletierten Fragmentes zu setzen, wobei die flankierenden Sequenzen die nachweisbare in vivo-Rekombination fördern;
b) das Vereinigen eines virulenten Vibrio cholerae-Stammes mit einem ersten Mikroorganismus, der das erste Plasmid trägt;
c) das Auswählen von Vibrio cholerae, die den ersten selektierbaren Marker exprimieren;
d) das Vereinigen des ausgewählten Produktes von Schritt c) mit einem zweiten Mikroorganismus, der ein zweites Plasmid mit einem zweiten selektierbaren Marker trägt, wobei das zweite Plasmid mit dem ersten Plasmid inkompatibel ist;
e) das Auswählen von Vibrio cholerae, die sowohl den ersten selektierbaren Marker als auch den zweiten selektierbaren Marker exprimieren;
f) das Erhalten eines dritten Plasmides, das flankierende Vibrio cholerae-Sequenzen von einem oder mehreren deletierten DNA-Fragmenten umfaßt, die den in Schritt a) beschriebenen homolog sind, sich jedoch durch die Abwesenheit eines selektierbaren Marker fremden Ursprungs unterscheiden;
g) das Vereinigen des ausgewählten Produktes von Schritt e) mit einem dritten Mikroorganismus, der das dritte Plasmid trägt; und
h) das Auswählen von Vibrio cholerae, die den ersten selektierbaren Marker nicht länger exprimieren.

11. Verfahren zum Isolieren von Deletionsmutanten von Vibrio cholerae gemäß einem der Ansprüche 6 bis 10, wobei die Deletion in dem ctx-Gen durch XbaI- und ClaI-Restriktionsendonukleaseschnittstellen definiert ist und die Deletion im zot-Gen durch StuI- und AccI-Restriktionsendonukleaseschnittstellen definiert ist.

12. Vibrio cholerae-Kultur gemäß einem der Ansprüche 1 bis 5, bei der das erste deletierte DNA-Fragment aus den Genen der A-und der B-Untereinheit des Vibrio cholerae-Toxines deletiert ist.

13. Vibrio cholerae-Kultur gemäß Anspruch 12, worin der Vibrio cholerae-Stamm ein Quecksilberresistenzgen hat und eine für die B-Untereinheit des Vibrio cholerae-Toxins kodierende DNA in das Chromosom des Vibrio cholerae insertiert ist.

14. Vibrio cholerae-Kultur nach Anspruch 13, wobei das Quecksilberresistenzgen und die für die B-Untereinheit von Vibrio cholerae-Toxin kodierende DNA an der Stelle eines Hämolysingenes im Vibrio cholerae-Chromosom insertiert sind.

15. Vibrio cholerae-Kultur nach Anspruch 14, wobei die Kultur Vibrio cholerae CVD 110 (ATTC Nr. 55,188) umfaßt.

16. Verfahren zum Isolieren von Deletionsmutanten von Vibrio cholerae, bei denen ein Bereich chromosomaler DNA, der für Cholera-Toxin und Zonula-occludens-Toxin kodiert, deletiert ist, und bei dem ein Quecksilberresistenzgen und eine für die B-Untereinheit von Vibrio cholerae-Toxin kodierende DNA insertiert ist, umfassend:
a) das Erhalten eines Plasmides, das Vibrio cholerae-DNA-Sequenzen, die für Cholera-Toxin und Zonula-occludens-Toxin kodieren, und ein Gen für einen selektierbaren Marker fremden Ursprungs umfaßt, wobei das Plasmid in Vibrio cholerae nicht extrachromosomal replizieren kann;
b) das Vereinen eines Mikroorganismus, der das Plasmid trägt, mit einem virulenten Vibrio cholerae-Stamm, der die für Cholera-Toxin und Zonula-occludens-Toxin kodierenden Sequenzen zwischen flankierenden Kopien einer zweiten Sequenz insertiert enthält, was die nachweisbare in vivo-Rekombination fördert;
c) das Auswählen von Vibrio cholerae, die den selektierbaren Marker exprimieren;
d) das Wachsenlassen des ausgewählten Produktes von Schritt (c) in Abwesenheit eines selektiven Agenzes;
e) das Auswählen von Vibrio cholerae, die den selektiven Marker nicht länger exprimieren;
f) das Erhalten eines zweiten Plasmides, das ein Quecksilberresistenzgen und eine für die B-Untereinheit des Vibrio cholerae-Toxines kodierende DNA und ein Gen für einen zweiten selektierbaren Marker fremden Ursprungs enthält, wobei das zweite Plasmid in Vibrio cholerae nicht extrachromosomal replizieren kann, und wo Sequenzen, die nachweisbare in vivo-Rekombination fördern, das Quecksilberresistenzgen und die für die B-Untereinheit des Vibrio cholerae-Toxins kodierende DNA flankieren;
g) das Vereinen eines Mikroorganismus, der das zweite Plasmid trägt, mit Vibrio cholerae gemäß Schritt e);
h) das Auswählen von Vibrio cholerae, die den zweiten selektierbaren Marker exprimieren;
i) das Wachsenlassen des ausgewählten Produktes von Schritt h) in Abwesenheit eines zweiten selektiven Markers;
j) das Auswählen von Vibrio cholerae, die den zweiten selektiven Marker nicht länger exprimieren; und
k) das Durchmustern von in Schritt j) erwähnten Vibrio cholerae auf Vibrio cholerae, die ein Quecksilberresistenzgen und für die B-Untereinheit von Vibrio cholerae-Toxin kodierende DNA enthalten und bei denen ein Bereich chromosomaler DNA, der für Cholera-Toxin und Zonula-occludens-Toxin kodiert, deletiert ist.

17. Verfahren zum Isolieren von Deletionsmutanten von Vibrio cholerae gemäß Anspruch 16, wobei das zweite Plasmid pCVD662.2B (ATTC NR. 68,630) ist.

18. Verfahren zum Isolieren von Deletionsmutanten von Vibrio cholerae gemäß Anspruch 16 oder 17, worin die flankierenden Sequenzen Hemolysingensequenzen enthalten.

19. Verfahren zum Isolieren von Deletionsmutanten von Vibrio cholerae gemäß Anspruch 16, wobei der in Schritt e) erwähnte Vibrio cholerae Vibrio cholerae CVD 109 (ATCC Nr. 55,057) ist.

20. Verfahren zum Isolieren von Deletionsmutanten von Vibrio cholerae gemäß einem der Ansprüche 16 bis 19, worin der in Schritt j) ausgewählte Vibrio cholerae Vibrio cholerae CVD 110 (ATCC Nr. 55,188) ist.

21. Verfahren zum Isolieren von Deletionsmutanten von Vibrio cholerae gemäß einem der Ansprüche 16 bis 19, worin der selektierbare Marker und der zweite selektierbare Marker Ampicillinresistenz sind.

22. Verfahren zum Isolieren von Deletionsmutanten von Vibrio cholerae gemäß einem der Ansprüche 16 bis 19, worin der selektierbare Marker Ampicillinresistenz und der zweite selektierbare Marker Chloramphenicolresistenz ist.

23. Vibrio cholerae CVD 110 (ATCC Nr. 55,188).

24. Verfahren zum Herstellen eines Impfstoffes zum Schützen gegen die Symptome von Cholera, umfassend das Vereinen eines Vibrio cholerae-Stammes des Ogawa- oder Inaba-Serotyps, bei dem ein erstes DNA-Fragment, das für das Vibrio cholerae-Toxin oder die A₁-Untereinheit davon kodiert, deletiert ist, um Avirulenz zu verleihen, und der die Fähigkeit zur Besiedelung des Darmes eines Wirtstieres beibehält, und bei dem ein zweites DNA-Fragment, das für das Zonula-occludens-Toxin oder ein Fragment davon kodiert, deletiert ist, mit einem pharmazeutisch verträglichen Verdünnungsmittel.

25. Verfahren nach Anspruch 24, wobei der Vibrio cholerae Vibrio cholerae CVD 110 (ATCC Nr. 55, 188) ist.

26. Verfahren nach Anspruch 24, wobei das erste deletierte DNA-Fragment für einen Teil des Vibrio cholerae-Toxines kodiert.

27. Verfahren nach Anspruch 24, wobei das erste DNA-Fragment die A₁-Untereinheit des ctx-Genes umfaßt.

28. Verfahren nach Anspruch 24, wobei das erste deletierte DNA-Fragment aus den Genen für die A-Untereinheit und die B-Untereinheit des Vibrio cholerae-Toxins deletiert ist.

29. Verfahren nach Anspruch 28, wobei der Vibrio cholerae-Stamm ein Quecksilberresistenzgen hat und eine DNA, die für die B-Untereinheit von Vibrio cholerae kodiert.

30. Verfahren nach Anspruch 29, wobei das Quecksilberresistenzgen und die für die B-Untereinheit kodierende DNA des Vibrio cholerae-Toxines an der Stelle des Hämolysingenes im Vibrio cholerae-Chromosom insertiert sind.

## Revendications (Revendications pour l'(les) Etat(s) contractant(s) suivant(s): AT, BE, CH, DE, DK, FR, GB, IT, LI, LU, NL, SE)

1. Culture de *Vibrio cholerae*, comprenant une souche de *Vibrio cholerae* du sérotype Ogawa ou Inaba dont un premier fragment d'ADN codant pour la toxine de *Vibrio cholerae* ou sa sous-unité A₁, est supprimé pour conférer l'avirulence, et conservant la capacité de coloniser l'intestin d'un animal-hôte, et donc un deuxième fragment d'ADN codant pour la toxine de *zonula occludens* ou un fragment de celle-ci, est supprimé pour recuire les effets secondaires résiduels dans ledit animal-hôte.

2. Culture *de Vibrio cholerae* selon la revendication 1, dans laquelle ledit premier fragment d'ADN supprimé, code pour une partie de la toxine de *Vibrio cholerae.*

3. Culture de *Vibrio cholerae* selon la revendication 1 ou 2, dans laquelle ledit premier fragment d'ADN comprend la sous-unité A₁ du gène ctx.

4. Culture de *Vibrio cholerae* selon l'une quelconque des revendications 1 à 3, dans laquelle ladite culture est utile pour la vaccination contre le choléra.

5. Culture de *Vibrio cholerae* comprenant un *Vibrio cholerae* du sérotype Ogawa ou Inaba, dont une région de l'ADN chromosomique codant pour la toxine cholérique et la toxine de *zonula occludens,* est supprimée pour conférer l'avirulence, et conserver la capacité de coloniser l'intestin d'un animal-hôte et pour réduire les effets secondaires résiduels dans ledit animal-hôte.

6. Procédé d'isolement de mutants de délétion de *Vibrio cholerae* donc une région de l'ADN chromosomique codant pour la toxine cholérique ou une partie de celle-ci, et pour la toxine de *zonula occludens,* est supprimée, selon lequel :
(a) on prépare un plasmide comprenant des séquences d'ADN de *Vibrio cholerae* codant pour la toxine cholérique et la toxine de *zonula occludens,* et un gène d'un marqueur de sélection d'origine étrangère, ledit plasmide étant incapable de se répliquer à l'extérieur du chromosome dans *Vibrio cholerae* ;
(b) on conjugue un micro-organisme comportant ledit plasmide, avec une souche virulente de *Vibrio cholerae* contentant lesdites séquences codant pour la toxine cholérique et la toxine de *zonula occludens,* insérées entre des copies adjacentes d'une deuxième séquence favorisant une recombinaison détectable in vivo ;
(c) on sélectionne *Vibrio cholerae* exprimant ledit marqueur de sélection ;
(d) on cultive le produit sélectionné de l'étape (c), en l'absence d'un agent sélectif ;
(e) on sélectionne *Vibrio cholerae* n'exprimant plus le marqueur sélectif.

7. Procédé d'isolement de mutants de délétion de *Vibrio cholerae* selon la revendication 6, dans lequel ladite deuxième séquence comprend un élément de séquence répétée (RS1) d'environ 2 700 paires de bases.

8. Procédé selon la revendication 6, dans lequel ledit plasmide est pCVD51 (ATCC N° 68 335).

9. Procédé selon la revendication 6, dans lequel ladite souche virulente est E7946 (ATCC N° 55 056).

10. Procédé d'isolement de mutants de délétion de *Vibrio cholerea*, comportant une délétion dans le gène ctx, ou une partie de celui-ci et une délétion dans le gène zot, selon lequel :
(a) on prépare un premier plasmide comprenant les séquences adjacentes de *Vibrio cholerae* d'un ou plusieurs fragments d'ADN supprimés, et un gène d'un marqueur de sélection d'origine étrangère, lié auxdites séquences adjacentes, afin de remplacer et d'être à la place du fragment supprimé, lesdites séquences adjacentes favorisant une recombinaison détectable in vivo ;
(b) on conjugue une souche virulente de *Vibrio cholerae,* avec un premier micro-organisme comportant le premier plasmide ;
(c) on sélectionne *Vibrio cholerae* exprimant ledit premier marqueur de sélection ;
(d) on conjugue le produit sélectionné de l'étape (c) avec un deuxième micro-organisme comportant un deuxième plasmide doté d'un deuxième marqueur de sélection, ledit deuxième plasmide étant incompatible avec le premier plasmide ;
(e) on sélectionne *Vibrio cholerae* exprimant tant le premier marqueur de sélection que le deuxième marqueur de sélection ;
(f) on prépare un troisième plasmide comprenant les séquences adjacentes de *Vibrio cholerae* à un ou plusieurs fragments d'ADN supprimés, homologues de celles décrites dans l'étape (a) mais différent par l'absence d'un marqueur de sélection d'origine étrangère ;
(g) on conjugue le produit de sélection de l'étape (e) avec un troisième micro-organisme comportant le troisième plasmide ; et
(h) on sélectionne *Vibrio cholerae* n'exprimant plus le premier marqueur de sélection.

11. Procédé d'isolement de mutants de délétion de *Vibrio cholerae* selon l'une quelconque des revendications 6 à 10, dans lequel ladite délétion. dans le gène ctx, est délimitée par des sites des endonucléases de restriction XbaI et ClaI, en ladite délétion dans le gène zot est délimitée par des sites d'endonucléases de restriction StuI et AccI.

12. Culture de *Vibrio cholerae* selon l'une quelconque des revendications 1 à 5, dans laquelle le premier fragment d'ADN supprimé est supprimé dans les gènes des sous-unités A et B de la toxine de *Vibrio cholerae.*

13. Culture de *Vibrio cholerae* selon la revendication 12, dans laquelle ladite souche ce *Vibrio cholerae* comprend un gène de résistance au mercure, et un ADN codant pour la sous-unité B de la toxine de *Vibrio cholerae,* insérés dans le chromosome de *Vibrio cholerae.*

14. Culture de *Vibrio cholerae* selon la revendication 13, dans laquelle ledit gène de résistance au mercure et l'ADN codant pour la sous-unité B de la toxine de *Vibrio cholerae,* sont insérés au site d'un gène d'hémolysine dans le chromosome de *Vibrio cholerae.*

15. Culture de *Vibrio cholerae* selon la revendication 14, dans laquelle ladite culture comprend *Vibrio cholerae* CVD 110 (ATCC N° 55 188).

16. Procédé d'isolement de mutants de délétion de *Vibrio cholerae,* dont une région d'ADN chromosomique codant pour la toxine cholérique et la toxine de *zonula occludens,* est supprimée, et dans lesquels sont insérés un gène de résistance eu mercure et un ADN codant pour la sous-unité B de la toxine de *Vibrio cholerae,* selon lequel :
(a) on prépare un plasmide comprenant des séquences de *Vibrio cholerae* codant pour la toxine cholérique et la toxine de *zonula occludens,* et un gène d'un marqueur de sélection d'origine étrangère, ledit plasmide étant incapable de se répliquer à l'extérieur du chromosome dans *Vibrio cholerae ;*
(b) on conjugue un micro-organisme comportant ledit plasmide avec une souche virulente de *Vibrio* cholerae contenant lesdites séquences codant pour la toxine cholérique et la toxine de *zonula occludens,* insérées entre des copies adjacentes d'une deuxième séquence favorisant une recombinaison détectable in vivo ;
(c) on sélectionne *Vibrio cholerae* exprimant ledit marqueur de sélection ;
(d) on cultive le produit sélectionné de l'étape (c) en l'absence d'un agent sélectif ;
(e) on sélectionne *Vibrio cholerae* n'exprimant plus le marqueur sélectionnable ;
(f) on prépare un deuxième plasmide comprenant un gène de résistance au mercure et un ADN codant pour la sous-unité B de la toxine de *Vibrio cholerae*, et un gêne d'un deuxième marqueur de sélection d'origine étrangère, ledit deuxième plasmide étant incapable de se répliquer à l'extérieur du chromosome dans *Vibrio cholerae,* et dans lequel, les séquences favorisant une recombinaison détectable in vivo, sont adjacentes audit gène de résistance au mercure et à l'ADN codant pour la sous-unité B de la toxine de *Vibrio cholerae ;*
(g) on conjugue un micro-organisme comportant ledit deuxième plasmide avec ledit *Vibrio cholerae* mentionné dans l'étape (e)
(h) on sélectionne *Vibrio cholerae* exprimant ledit deuxième marqueur de sélection ;
(i) on cultive le produit sélectionné de l'étape (h) en l'absence d'un deuxième agent sélectif ;
(j) on sélectionne *Vibrio cholerae* n'exprimant plus ledit deuxième marqueur sélectif ; et
(k) on crible ledit *Vibrio cholerae* mentionné dans l'étape (j) eu égard à *Vibrio cholerae* comportant un gène de résistance au mercure et un ADN codant pour la sous-unité de la toxine de *Vibrio cholerae,* et dont une région l'ADN chromosomique codant pour la toxine cholérique et la toxine de *zonula occludens,* est supprimée.

17. Procédé d'isolement de mutants de délétion de *Vibrio cholerae* selon la revendication 16, dans lequel ledit deuxième plasmide est pCVD662.2B (ATCC N° 68 630).

18. Procédé d'isolement de mutants de délétion de *Vibrio cholerae* selon la revendication 16 ou 17, dans lequel lesdites séquences adjacentes comprennent des séquences de gêne d'hémolysine.

19. Procédé d'isolement de mutants de délétion de *Vibrio cholerae* selon la revendication 16, dans lequel ledit *Vibrio cholerae* mentionné dans l'étape (e), est *Vibrio chnolerae* CVD 109 (ATCC N° 55 057).

20. Procédé d'isolement de mutants de délétion de *Vibrio cholerae* selon l'une quelconque des revendications 16 à 19, dans lequel ledit *Vibrio cholerae* sélectionné dans l'étape (j) est *Vibrio cholerae* CVD 110 (ATCC N° 55 188).

21. Procédé d'isolement de mutants de délétion de *Vibrio cholerae* selon l'une quelconque des revendications 16 à 19, dans lequel ledit marqueur de sélection en ledit deuxième marqueur de sélection, sont la résistance à l'ampicilline.

22. Procédé d'isolement des mutants de délétion de *Vibrio cholerae* selon l'une quelconque des revendications 16 à 19, dans lequel ledit marqueur de sélection est la résistance à l'ampicilline, et ledit deuxième marqueur sélectionnable est la résistance au chloramphénicol.

23. *Vibrio cholerae* CVD 110 (ATCC N° 55 188).

24. Vaccin de protection contre les symptômes du choléra, comprenant une souche de *Vibrio cholerae* du sérotype Ogawa ou Inaba dont un premier fragment d'ADN codant pour la toxine de *Vibrio cholerae* ou sa sous-unité A₁, est supprimé pour conférer l'avirulence,
et conservant la capacité de coloniser l'intestin d'un animal-hôte, et dont un deuxième fragment d'ADN codant pour la toxine de *zonula occludens* ou d'un fragment de celle-ci, est supprimé.

25. Vaccin selon la revendication 24, dans lequel ledit *Vibrio cholerae* est *Vibrio cholerae* CVD 110 (ATCC N° 55 188).

## Revendications (Revendications pour l'(les) Etat(s) contractant(s) suivant(s): GR, ES)

1. Culture de *Vibrio cholerae* comprenant une souche de *Vibrio cholerea* du sérotype Ogawa ou Inaba, dont un premier fragment d'ADN codant pour la toxine de *Vibrio cholerea* ou la sous-unité A₁ de celle-ci, est supprimé pour conférer l'avirulence, et conservant la capacité de coloniser l'intestin d'un animal-hôte, en dont un deuxième fragment d'ADN codant pour la Toxine de *zonula occludens* ou d'un fragment de celle-ci, est supprimé pour réduire les effets secondaires résiduels dans ledit animal-hôte.

2. Culture ce *Vibrio cholerae* selon la revendication 1, dans laquelle ledit premier fragment d'ADN supprimé, code pour une partie de la toxine de *Vibrio cholerae.*

3. Culture de *Vibrio cholerae* selon la revendication 1 ou 2, dans laquelle ledit premier fragment d'ADN comprend la sous-unité A₁ du gène ctx.

4. Culture de *Vibrio cholerae* selon l'une quelconque des revendications 1 à 3, dans laquelle ladite culture est utile pour la vaccination contre le choléra.

5. Culture de *Vibrio cholerae* comprenant un *Vibrio cholerae* du sérotype Ogawa ou Inaba, dont une région de l'ADN chromosomique codant pour la toxine cholérique et la toxine de *zonula occludens,* est supprimée pour conférer l'avirulence et conserver la capacité de coloniser l'intestin d'un animal-hôte et pour réduire les effets secondaires résiduels dans ledit animal-hôte.

6. Procédé d'isolement de mutants de délétion de *Vibrio cholerae,* dont une région de l'ADN chromosomique codant pour la toxine cholérique ou une partie de celle-ci, et pour la toxine de *zonula occludens,* est supprimée, selon lequel :
(a) on prépare un plasmide comprenant des séquences d'ADN de *Vibrio cholerae* codant pour la toxine cholérique et la toxine de *zonula occludens,* et un gène d'un marqueur de sélection d'origine étrangère, ledit plasmide étant incapable de se répliquer à l'extérieur du chromosome dans *Vibrio cholerae ;*
(b) on conjugue un micro-organisme comportant ledit plasmide, avec une souche virulente de *Vibrio cholerae* contenant lesdites séquences codant pour la toxine cholérique et la toxine de *zonula occludens,* insérées entre des copies adjacentes d'une deuxième séquence favorisant une recombinaison détectable in vivo ;
(c) on sélectionne *Vibrio cholerae* exprimant ledit marqueur de sélection ;
(d) on cultive le produit sélectionné de l'étape (c), en l'absence d'un agent sélectif ;
(e) on sélectionne *Vibrio cholerae* n'exprimant plus le marqueur sélectif.

7. Procédé d'isolement de mutants de délétion de *Vibrio cholerae* selon la revendication 6, ladite deuxième séquence comprenant un élément de séquence répétée (RS1) d'environ 2 700 paires de bases.

8. Procédé selon la revendication 6, dans lequel ledit plasmide est pCVD51 (ATCC N° 68 335).

9. Procédé selon la revendication 6, dans lequel ladite souche virulente est E7946 (ATCC N° 55 056).

10. Procédé d'isolement de mutants de délétion de *Vibrio cholerae* comportant une délétion dans le gène ctx, ou une partie de celui-ci, et une délétion dans le gène zot, selon lequel :
(a) on prépare un premier plasmide comprenant des séquences adjacentes de *Vibrio cholerae* à au moins un fragment d'ADN supprimé, et un gène d'un marqueur de sélection d'origine étrangère, lié auxdites séquences adjacentes, pour remplacer et d'être à la place de ce fragment supprimé, lesdites séquences adjacentes favorisant une recombinaison détectable in vivo ;
(b) on conjugue une souche virulente de *Vibrio cholerae,* avec un premier micro-organisme comportant le premier plasmide ;
(c) on sélectionne *Vibrio cholerae* exprimant ledit premier marqueur de sélection ;
(d) on conjugue le produit sélectionné de l'étape (c) avec un deuxième micro-organisme comportant un deuxième plasmide doté d'un deuxième marqueur de sélection, ledit deuxième plasmide étant incompatible avec le premier plasmide ;
(e) on sélectionne *Vibrio cholerae* exprimant tant le premier marqueur de sélection que le deuxième marqueur de sélection ;
(f) on prépare un troisième plasmide comprenant les séquences adjacentes de *Vibrio cholerae* à un ou plusieurs fragments d'ADN supprimés, homologues de celui décrit dans l'étape (a) mais différent par l'absence d'un marqueur de sélection d'origine étrangère ;
(g) on conjugue le produit sélectionné de l'étape (e) avec un troisième micro-organisme comportant le troisième plasmide ; en
(h) on sélectionne *Vibrio cholerae* n'exprimant plus le premier marqueur de sélection.

11. Procédé d'isolement de mutants de délétion de *Vibrio cholerae* selon l'une quelconque des revendications 6 à 10, dans lequel ladite délétion dans le gène ctx est délimitée par des sites des endonucléases de restriction XbaI et ClaI, et ladite délétion dans le gêne zot est délimitée par des sites des endonucléases de restriction StuI et AccI.

12. Culture de *Vibrio cholerae* selon l'une quelconque des revendications 1 à 5, dans laquelle le premier fragment d'ADN supprimé est supprimé dans les gènes des sous-unités A et B de la toxine de *Vibrio cholerae.*

13. Culture de *Vibrio cholerae* selon la revendication 12, dans laquelle ladite souche de *Vibrio cholerae* comprend un gène de résistance au mercure et un ADN codant pour la sous-unité B de la toxine de *Vibrio cholerae,* insérés dans le chromosome de *Vibrio cholerae.*

14. Culture de *Vibrio cholerae* selon la revendication 13, dans laquelle ledit gène de résistance au mercure et l'ADN codant pour la sous-unité B de la toxine de *Vibrio cholerae,* sont insérés au site d'un gène d'hémolysine dans le chromosome de *Vibrio cholerae.*

15. Culture de *Vibrio cholerae* selon la revendication 14, dans laquelle ladite culture comprend *Vibrio cholerae* CVD 110 (ATCC N° 55 188).

16. Procédé d'isolement de mutants de délétion de *Vibrio cholerae* dont une région d'ADN chromosomique codant pour la toxine cholérique et la toxine de *zonula occludens,* est supprimée, et dans lesquels sont insérés un gène de résistance au mercure et un ADN codant pour la sous-unité B de la toxine de *Vibrio cholerae,* selon lequel :
(a) on prépare un plasmide comprenant des séquences de *Vibrio cholerae* codant pour la toxine cholérique et la toxine de *zonula occludens,* et un gène d'un marqueur de sélection d'origine étrangère, ledit plasmide étant incapable de se répliquer à l'extérieur des chromosomes dans *Vibrio cholerae ;*
(b) on conjugue un micro-organisme comportant ledit plasmide avec une souche virulente de *Vibrio* cholerae contenant les séquences codant pour la toxine cholérique et la toxine de *zonula occludens,* insérées entre des copies adjacentes d'une deuxième séquence favorisant une recombinaison détectable in vivo ;
(c) on sélectionne *Vibrio cholerae* exprimant ledit marqueur de sélection ;
(d) on cultive le produit sélectionné de l'étape (c) en l'absence d'un agent sélectif ;
(e) on sélectionne *Vibrio cholerae* n'exprimant plus le marqueur sélectif ;
(f) on prépare un deuxième plasmide comprenant un gène de résistance au mercure et un ADN codant pour la sous-unité B de la toxine de *Vibrio cholerae,* et un gène d'un deuxième marqueur de sélection d'origine étrangère, ledit deuxième plasmide étant incapable de se répliquer à l'extérieur des chromosomes dans *Vibrio cholerae,* et dans lequel les séquences favorisant une recombinaison détectable in vivo, sont adjacentes audit gène de résistance au mercure et l'ADN codant pour la sous-unité B de la toxine de *Vibrio cholerae ;*
(g) on conjugue un micro-organisme comportant ledit deuxième plasmide avec ledit *Vibrio cholerae* mentionné dans l'étape (e) ;
(h) on sélectionne *Vibrio cholerae* exprimant ledit deuxième marqueur de sélection ;
(i) on cultive le produit sélectionné de l'étape (h) en l'absence d'un deuxième agent sélectif ;
(j) on sélectionne *Vibrio cholerae n'exprimant* plus ledit deuxième marqueur sélectif ; et
(k) on crible ledit *Vibrio cholerae* mentionné dans l'étape (j) eu égard à *Vibrio cholerae* comportant un gène de résistance au mercure et un ADN codant pour la sous-unité de la toxine de *Vibrio cholerae,* et dont une région d'ADN chromosomique codant pour la toxine cholérique et la toxine de *zonula occludens,* est supprimée.

17. Procédé d'isolement de mutants de délétion de *Vibrio cholerae* selon la revendication 16, dans lequel ledit deuxième plasmide est pCVD662.2B (ATCC N° 68 630).

18. Procédé d'isolement de mutants de délétion de *Vibrio cholerae* selon la revendication 16 ou 17, dans lequel lesdites séquences adjacentes comprennent des séquences de gène d'hémolysine.

19. Procédé d'isolement de mutants de délétion de *Vibrio cholerae* selon la revendication 16, dans lequel ledit *Vibrio cholerae* de l'étape (e), est *Vibrio cholerae* CVD 109 (ATCC N° 55 057).

20. Procédé d'isolement de mutants de délétion de *Vibrio cholerae* selon l'une quelconque des revendications 16 à 19, dans lequel ledit *Vibrio cholerae* sélectionné dans l'étape (j), est le *Vibrio cholerae* CVD 110 (ATCC N° 55 188).

21. Procédé d'isolement de mutants de délétion de *Vibrio cholerae* selon l'une quelconque des revendications 16 à 19, dans lequel ledit marqueur de sélection et ledit deuxième marqueur de sélection, sont la résistance à l'ampicilline.

22. Procédé d'isolement des mutants de délétion de *Vibrio cholerae* selon l'une quelconque des revendications 16 à 19, dans lequel ledit marqueur de sélection est la résistance à l'ampicilline, et ledit deuxième marqueur de sélection est la résistance au chloramphénicol.

23. *Vibrio cholerae* CVD 110 (ATCC N° 55 188).

24. Procédé de préparation d'un vaccin de protection contre les symptômes du choléra, selon lequel on associe avec un diluant pharmaceutiquement acceptable, une souche de *Vibrio cholerae* du sérotype Ogawa ou Inaba, ladite souche comportant un premier fragment d'ADN codant pour la toxine de *Vibrio cholerae* ou sa sous-unité A₁, supprimé pour conférer l'avirulence, et conservant la capacité de coloniser l'intestin d'un animal-hôte, et ayant un deuxième fragment d'ADN codant pour la toxine de *zonula occludens* ou un fragment de celle-ci, supprimé.

25. Procédé selon la revendication 24, dans lequel ledit *Vibrio cholerae* est *Vibrio cholerae* CVD 110 (ATCC N° 55 188).

26. Procédé selon la revendication 24, dans lequel ledit premier fragment d'ADN supprimé code pour une partie de la toxine de *Vibrio cholerae.*

27. Procédé selon la revendication 24, dans lequel le premier fragment d'ADN comprend la sous-unité A₁ du gène ctx.

28. Procédé selon la revendication 24, dans lequel le premier fragment d'ADN est supprimé dans les gènes des sous-unités A et B de la toxine de *Vibrio cholerae.*

29. Procédé selon la revendication 28, dans lequel ladite souche de *Vibrio cholerae* comprend un gène de résistance au mercure et un ADN codant pour la sous-unité B du *Vibrio cholerae.*

30. Procédé selon la revendication 29, dans lequel ledit gène de la résistance au mercure et ledit ADN codant pour la sous-unité B de la toxine de *Vibrio cholerae,* sont insérés au site d'un gène d'hémolysine dans le chromosome de *Vibrio cholerae.*
